(19) Europäisches Patentamt
European Patent Office
Office européen des brevets

(11) Publication number: **0 078 247**
**B1**

(12) **EUROPEAN PATENT SPECIFICATION**

(45) Date of publication of patent specification: **27.11.85**

(51) Int. Cl.$^4$: **B 01 J 27/02,** C 07 C 76/02, C 07 C 79/00, C 07 B 43/02

(21) Application number: **82870055.9**

(22) Date of filing: **19.10.82**

(54) **Process and catalysts for vapor phase nitration of aromatic compounds.**

(30) Priority: **21.10.81 US 313522**
**21.10.81 US 313519**

(43) Date of publication of application:
**04.05.83 Bulletin 83/18**

(45) Publication of the grant of the patent:
**27.11.85 Bulletin 85/48**

(84) Designated Contracting States:
**DE FR GB IT**

(56) References cited:
**EP-A-0 014 052**
**DE-A-2 234 328**
**DE-A-2 401 958**
**DE-A-2 510 095**
**FR-A-2 358 381**
**GB-A-2 000 141**
**US-A-3 914 332**
**US-A-4 107 220**

(73) Proprietor: **MONSANTO COMPANY**
**Patent Department 800 North Lindbergh Boulevard**
**St. Louis, Missouri 63166 (US)**

(72) Inventor: **Schumacher, Ignatius**
**336 West Clayton Road**
**Ballwin Missouri 63011 (US)**
Inventor: **Wang, Kang-bo**
**13386 Primwood Drive**
**Creve Coeur Missouri 63141 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 078 247 B1

**Description**

This invention relates to a process for the vapor phase nitration of aromatic compounds in the presence of catalyst compositions, some of which are new.

The invention also relates to the novel catalyst compositions. Such compositions may be used for any of a wide variety of purposes generally known in the art. Thus, for example, the compositions are useful as catalysts in the transformation of numerous organic compounds in the vapor phase such as alkylation and/or nitration of aromatic compounds, dehydrogenation reactions, oxidation reactions, hydrogenations reactions, skeletal isomerization reactions, and the like. The catalyst compositions may be employed in a manner identical to that for catalysts heretofore known in the art for such transformation.

Description of the prior art

Various vapor processes which employ numerous catalyst compositions are known.

For example, EP—A—0 014 052 discloses a catalytic material for the oxidation of sulfur dioxide to sulfur trioxide, comprising a catalyst which includes sulfatized vanadium ions as the active catalytic component, and two different promoters, the first of which comprises cesium ions, optionally with ions of another alkali metal, and a second promoter comprising ions of a metal such as aluminium, magnesium, yttrium or lanthanum.

DE—A—2 401 958 refers to a catalyst prepared by passing a stream of $SO_3$ through silica gel, reported to be useful for the vapor phase hydration of olefins.

US—A—3 914 332 discloses a process for the oxidative dehydrogenation of butane to a mixture of 1- and 2-butenes and butadiene using a catalyst comprising $V_2O_5$, $K_2SO_4$, $SO_3$ and $SiO_2$.

The vapor phase nitration of benzene and toluene in the presence of silica gel at temperatures ranging from about 275°C, to about 310°C. is described in McKee and Wilhelm, *Industrial and Engineering Chemistry, 28*(6), 662—667 (1936) and U.S. Patent 2,109,873. Bauxite and alumina were reported to be ineffective as catalysts in the vapor phase nitration of benzene.

U.S. Patent 4,107,220 discloses the use of molecular sieves (aluminosilicates) having a pore size varying from about 5 Å to about 10 Å as catalysts to control the para-to-ortho isomer distribution of nitrochlorobenzene in the vapor phase nitration of chlorobenzene. Suitable temperatures are reported to range from about 190°C. to about 290°C.

The use of silver-containing catalysts in processes for the direct oxidation of ethylene to ethylene oxide is disclosed in numerous patents, for example, U.S. Patents 3,962,136, 3,793,231, 2,831,870 to cite just a few.

Crystalline alumina silicate is reported in Harper et al, "Alkylation of Benzene with Propylene over a Crystalline Alumina Silicate," *ACS Symposium on Recent Advances in Alkylation Chemistry,* New Orleans, Louisiana, March 20—25 (1979) to be an effective catalyst in the alkylation of benzene with propylene to produce cumene and minor amounts of poly-isopropylbenzenes.

The vapor phase Friedel-Crafts alkylation of toluene and phenol with alkyl chloroformates and alkyl oxalates in the presence of a solid superacid, perfluorinated resinsulfonic acid, known as Nafion® —H, as catalyst is described in Olah et al, *Journal of Catalysis, 61*(1), 96—102 (1980). Such solid acids are designated as solid superacids due to their high acid strength, that is, having an acid strength stronger than 100 percent sulfuric acid. Similar alkylations of benzene with ethylene and propylene are disclosed in Olah et al, *Journal of Organic Chemistry, 42*(26), 4187—4191 (1977).

Solid superacids also are disclosed as effective to catalyze the skeletal isomerization of butane to isobutane and minor amounts of by-products. Among the solid superacids disclosed are zirconia/sulfate ion [Hino et al, *JCS Chemical Communications,* 851—852 (1980)], titania/sulfate ion [*Idem, ibid* 1148—1149 (1979)], iron (III) oxide/sulfate ion [Hino et al, *Chemistry Letters* (Japan) 1259—1260 (1979)], and $SbF_5$—$TiO_2$—$SiO_2$, $SbF_5$—$TiO_2$, and $SbF_5$—$SiO_2$—$Al_2O_3$ [Tanabe et al, *Chemistry Letters* (Japan), 625—626 (1979)].

Although these prior art catalysts are effective to provide the desired product, the commercial utility of a catalyst system is highly dependent upon the cost of the system, the conversion of the reactant(s) and the yield of the desired product(s). In many cases, a reduction in the cost of a catalyst system on the order of a few cents per pound or a small percent increase in the yield of the desired product represents a tremendous commercial economical savings. Accordingly, research efforts are continually being made to define new or improved catalyst systems and methods and processes of making new and old catalyst systems to reduce the cost and/or upgrade the activity and selectivity of such catalyst systems in particular processes. The discovery of the catalyst compositions of the present invention, therefore, is believed to be a decided advance in the catalyst art.

Nitroaromatic compounds are currently produced primarily via liquid phase reactions employing mixed acids. A sulfuric acid/nitric acid mixture is the most commonly employed industrial nitrating agent. Other mixed acids for nitration of aromatic compounds are acetic acid/nitric acid mixtures as described, for example, in U.S. Patent 3,180,900. In U.S. Patent 3,928,476, the latter type nitration is conducted over silica-alumina or alumina supports. A sulfonic acid/-nitric acid mixture is disclosed as a nitrating agent for the nitration of halobenzenes in U.S. Patent 3,077,502. Reportedly, the sulfonic acid causes a para directive effect, the effect of which is to increase the para-to-ortho isomer distribution above the usual ratio of 1.7.

Although these prior art processes generally provide the desired product, the choice of available catalysts is severely limited. In addition, the commercial utility of a catalytic process is highly dependent upon the cost of the catalyst employed, the conversion of the reactant(s), and the yield of the desired product(s). In many cases, a reduction in the cost of the catalyst system employed in a given process on the order of a few cents per pound or a small percent increase in the yield of the desired product represents a tremendous commercial economical savings. Thus, the discovery that the vapor phase nitration reaction of the present invention can be carried out in a very efficient manner with high aromatic compound conversion and high nitroaromatic compound selectivity is believed to be a decided advance in the art.

It is an object of this invention to provide a vapor phase nitration process for converting aromatic compounds to the corresponding nitroaromatic compounds characterized by high aromatic compound conversion and high nitroaromatic compound selectivity. This and other objects, aspects, and advantages of the invention will become apparent to those skilled in the art from the accompanying description and claims.

The above object is achieved by the improved process disclosed herein for the vapor phase nitration of aromatic compounds where the aromatic compound is contacted with a nitrating agent in the vapor phase to yield the corresponding nitroaromatic compound, characterised in that the nitration is conducted in the presence of water and a nitration promoting catalyst which comprises the adduct of:

(a) an alumina-silica-metal oxide combination represented by the formula:

$$(Al_2O_3)_a(SiO_2)_b(M_{2/n}O)_c$$

wherein M is a metal cation selected from the lanthanides or rare earths, Groups 1b, 2b, 5b, 6b, 7b, and 8 of the Periodic Table of the Elements, and mixtures thereof, and a, b, and c represents weight percent of the $Al_2O_3$, $SiO_2$, and $M_{2/n}O$ components, respectively, in the alumina-silica-metal oxide combination, with a being 0 to 100, b being 0 to 100, and c being 0 to 50, and n is an integer from 1 to 7 of the valence of the metal cation, with the proviso that the sum of (a+b) must be greater than 0, and

(b) a catalytically effective amount of sulfur trioxide.

In accordance with a further aspect of this invention, novel catalyst compositions are provided which comprise the adduct of:

(a) an alumina-silica-metal oxide combination represented by the formula:

$$(Al_2O_3)_a(SiO_2)_b(M_{2/n}O)_c$$

wherein M is a metal cation selected from the lanthanides or rare earths, Groups 1b, 2b, 5b, 6b, 7b, and 8 of the Periodic Table of the Elements, and mixtures thereof, and a, b, and c represent weight percent of the $Al_2O_3$, $SiO_2$, and $M_{2/n}O$ components, respectively, in the alumina-silica-metal oxide combination, with a being from greater than 0 to 100, b being 0 to 100, and c being 0 to 50, and n represents an integer from 1 to 7 of the valence of the metal cation, and

(b) a catalytically effective amount of sulfur trioxide, said catalyst compositions containing not more than 5 weight percent, based on the weight of the alumina-silica-metal oxide combination, of other substances.

Alumina-silica-metal oxide combination materials suitable for use in the present invention are those which yield the novel catalyst compositions of the present invention which are effective to catalyze the nitration of organic compounds in the vapor phase with high conversion of the reactants and high yields of desired products. Such materials may be crystalline, non-crystalline, or mixtures thereof. Nonlimiting representative examples of suitable alumina-silica-metal oxide combination materials are alumina (a=100; b=c=0), silica (b=100; a=c=0), alumina-silica, including aluminosilicates such as synthetic and naturally occurring zeolites, and mixtures thereof.

In many instances, it may be desirable to modify the physical and/or chemical properties of the alumina-silica-metal oxide combination or the spectrum of products produced in the many and varied reactions in which the compositions of the present invention are effective as catalysts. To this end, one of more metal oxide components may be incorporated into the alumina-silica-metal oxide combination. Depending upon the particular effect desired or property to be modified, suitable metal oxides are those wherein the metal cation (M) is selected from the group consisting of the lanthanides or rare earths, Groups 1b, 2b, 5b, 6b, 7b, and 8 of the Periodic Table of the Elements, and mixtures thereof. For example, adducts formed from the alumina-silica-metal oxide combinations containing oxides of vanadium, silver, copper, manganese, nickel, molybdenum, and tungsten may be employed for the production of olefin oxides from olefines. Adducts containing oxides of chromium, zinc, manganese, iron, copper, cerium, and cobalt may be employed for the production of olefinically unsaturated compounds, for example, propylene from propane, styrene from ethylbenzene, and the like. In general, metals and metal ions employed as catalysts in known prior art processes may be employed in the present invention in the same mode to effect similar reactions, but with the added advantage of enhanced activity.

The alumina-silica-metal oxide combination materials are available commercially from numerous catalyst suppliers. Such materials can also be prepared by procedures well known in the art. For example, the alumina-silica-metal oxide combination materials wherein c is greater than 0 can be prepared by known

procedures for preparing supported metal oxide catalysts. The method generally employed for producing such metal oxide catalysts involves impregnating the support—alumina, silica, and alumina-silica, for example—with a soluble metal salt convertible to the metal oxide, separating the saturated solid, and heating to remove a major portion of the solvent. The resultant material is then calcined to convert the metal salt to the corresponding metal oxide. In many cases, a multiple impregnation technique is employed to achieve a higher concentration of metal oxide on the support.

Another well-known technique involves suspending the support material in a solution of a metal salt convertible to the metal oxide, completely or partially evaporating the solvent, and possibly mixing of the resultant material with an organic binder and forming structures thereof. The dried structures are then heated to an elevated temperature to effect complete removal of solvent, burning out of the organic material, and as previously noted, conversion of the metal salt to the corresponding metal oxide.

The alumina-silica-metal oxide combination material need not necessarily be completely free of impurities. Thus, materials or substances other than $Al_2O_3$, $SiO_2$, and $M_{2/n}O$ which cause little, if any, adverse effect upon the resultant catalyst's activity may be present. Impurities which are commonly associated with alumina and alumina-containing materials include, for example, oxides of the alkali metals, the alkaline earth metals, and titanium of Group 4b of the Periodic Table of the Elements. In general, such impurities may be present in amounts not exceeding 5 weight percent.

The term Periodic Table of the Elements, as employed herein refers to the Periodic Table of the Elements published in *CRC Handbook of Chemistry and Physics,* 60th ed., Weast, Ed., CRC Press, Inc., Boca Raton, Florida, 1979, Inside Front Cover.

Sulfur trioxide ($SO_3$) is an essential component of the catalyst compositions of the present invention. It is added to the alumina-silica-metal oxide combination in a catalytically effective amount. It may be charged directly as sulfur trioxide in the vapor or gaseous phase. Alternatively, it may be provided indirectly by charging to the alumina-silica-metal oxide combination a mixture of sulfur dioxide ($SO_2$) and nitrogen dioxide ($NO_2$) which react to produce sulfur trioxide and inert (for purposes of the present invention) nitric oxide (NO). When a mixture of sulfur dioxide and nitrogen dioxide is employed, a stoichiometric mole ratio of at least 1 is required. It is preferred, however, to employ an excess of sulfur dioxide, usually on the order of about 2 to 3 moles per mole of nitrogen dioxide.

In general, when providing the sulfur trioxide, the indirect method of charging a mixture of sulfur dioxide and nitrogen dioxide to the alumina-silica-metal oxide combination is preferred in that both sulfur dioxide and nitrogen dioxide, as well as nitric oxide, exist in the gaseous state at ambient temperatures (approximately 25°C.) and above while sulfur trioxide exists as a liquid at ambient temperatures and under the usual and preferred preparative conditions would first have to be converted to a vapor prior to contacting the alumina-silica-metal oxide combination.

As previously indicated, the catalyst compositions of the present invention comprise an adduct, an essential component of which is sulfur trioxide. It is recognized, of course, that when a mixture of sulfur dioxide and nitrogen dioxide is charged to the reactor to provide the sulfur trioxide, the adsorbed species may in fact be a complex or combination of sulfur trioxide and nitrogen dioxide. However, regardless of the actual composition of the adsorbed species, it is conveniently referred to herein as sulfur trioxide and is meant to encompass all such compositions, whether sulfur trioxide, sulfur trioxide-nitrogen dioxide complex, or some combination thereof, as well as unreacted mixtures of sulfur dioxide and nitrogen dioxide.

The catalyst compositions of the present invention are normally prepared by contacting the alumina-silica-metal oxide combination with sulfur trioxide (directly or indirectly as previously described) in the vapor phase under conditions conducive to the formation of the adduct and for a time sufficient to induce the desired weight gain. The amount of added sulfur trioxide (as indicated by the gain in weight) is not narrowly critical. All that is necessary is that a catalytically effective amount of sulfur trioxide be added. In general, it has been found that at least 5 weight percent, based on the weight of the alumina-silica-metal oxide combination, sulfur trioxide is required to provide the enhanced activity exhibited by the catalyst compositions of the present invention. Also, although not critical, an upper limit of about 40 weight percent, with about 10 weight percent being preferred, has been found to be desireable in that little, if any, advantage is demonstrated for higher concentrations of sulfur trioxide. Thus, both higher and lower concentrations than the stated 5 to 40 weight percent range can be employed, if desired, but since such concentrations offer no particular advantage over the stated desirable range, and may in fact affect adversely the catalyst activity, particularly at concentrations less than about 5 weight percent, the stated 5 to 40 weight percent range is desirably employed.

The conditions under which the catalyst compositions are prepared can vary widely. All that is necessary is that the sulfur trioxide, whether charged directly or indirectly, exist in the vapor phase while contacting the alumina-silica-metal oxide combination. Thus, the catalyst preparation can be conducted at temperatures ranging from ambient temperatures (about 25°C). (when sulfur dioxide and nitrogen dioxide are employed to provide the sulfur trioxide) to about 300°C. or higher. Preferred temperatures, however, range from about 150°C. to about 250°C., with 175°C. to about 225°C. being particularly preferred. At such preferred temperatures, the uptake of sulfur trioxide is reasonably rapid with a minimum of loss of reactant gases resulting from unreacted pass-through. In general, and for convenience, the catalyst preparations

can be performed at the temperature to be employed in the subsequent reaction in which the catalyst is to be employed.

The catalyst preparations are conducted under substantially anhydrous conditions. This is necessary since sulfur trioxide readily undergoes reaction with water to form sulfuric acid which, prior to formation of the adducts comprising the catalyst compositions of the present invention, may exhibit an adverse effect in subsequent reactions. As employed herein, the term "substantially anhydrous" means not more than 5 weight percent water is present in the reaction as part of the catalyst-forming components.

The catalyst compositions of the present invention are conveniently prepared in an apparatus of the type suitable for carrying out chemical reaction in the vapor phase. In this manner the catalyst preparation can be performed in the same reactor as that to be employed for the subsequent reaction or transformation of organic compounds. It can be conducted in a fixed bed, moving bed, or a fluidized bed system to effect contacting of the alumina-silica-metal oxide combination and the sulfur trioxide. And, as previously noted, catalyst preparation is preferably carried out by continually passing a vaporous mixture of sulfur dioxide and nitrogen dioxide in a 2—3/l mole ratio over a bed of the alumina-silica-metal oxide combination under substantially anhydrous conditions at a temperature from about 25°C. to about 300°C., and usually, about 175°C. to about 225°C.

The vapor phase nitration process is characterized by high aromatic compound conversion and high nitroaromatic compound selectivity. And, in addition, when the aromatic compound starting material is a monosubstituted aromatic compound having an ortho-para orientation substituent, especially chlorobenzene, the observed para-to-ortho isomer distribution ranges from about 1.8—3.5/l, depending upon the particular nitration promoting catalyst and aromatic compound employed.

Aromatic compounds suitable for use in the present process are those which can exist in the vapor phase or state and undergo nitration under operating conditions to yield the desired nitroaromatic compounds. Moreover, in those instances where ortho and/or para isomers of the nitroaromatic compound are desired, the aromatic compound starting material must have an ortho- para orientation substituent such as halogen, lower alkyl, lower hydroxyalkyl, lower acetoxyalkyl, lower alkoxy, phenyl, and the like, where the term "lower alkyl" and related terms refer to substituents containing alkyl groups of 1 to 6 carbon atoms. Non-limiting representatives of suitable aromatic compounds include aromatic hydrocarbons, such as benzene, toluene, xylenes, ethylbenzene, cumene, naphthalene, and the like; aromatic ethers such as anisole, phenetole, and the like; halo-aromatic compounds such as chlorobenzene, bromobenzene, iodobenzene, o-dichlorobenzene, and the like; aromatic carboxylates such as benzoic acid, methyl benzoate, ethyl benzoate, and the like. It has been found, however, that the process of this invention is particularly efficacious with chlorobenzene (also known as monochlorobenzene or simply MCB), and benzene.

It will be apparent, of course, that mono-substituted aromatic compounds having an ortho-para orientation substituent—toluene and chlorobenzene, for example—upon being nitrated yield a nitroaromatic compound product containing ortho, meta, and para isomers. In such instances, the ortho and para isomers generally constitute the major portion of the product mixture, with the meta isomer being present in only trace amounts.

The nitrating agents which are employed in the process of this invention are the gaseous oxides of nitrogen higher than nitric oxide (NO) such as nitrogen dioxide ($NO_2$), dinitrogen trioxide ($N_2O_3$), and dinitrogen tetroxide ($N_2O_4$). Of these nitrating agents, nitrogen dioxide is preferred. Thus, for convenience and clarity, the process will be described with reference to the preferred nitrogen dioxide as the nitrating agent.

The vapor phase nitration process of this invention is not limited to a specific reaction temperature since the process can be conducted at temperatures ranging from about 80°C. to about 300°C. Preferred temperatures, however, range from about 150°C. to about 250°C., with 175°C. to about 225°C. being particularly preferred. At such preferred temperatures, the rate of reaction is reasonably rapid and little, if any, by-product formation occurs. It will be appreciated, however, that the particular temperature employed for a given aromatic compound will depend to some extent upon the boiling point or vaporization temperature of the particular aromatic compound. For example, when chlorobenzene, which has a boiling point of 132°C., is the aromatic compound of choice, the vapor phase nitration is conveniently carried out within the aforesaid preferred and most preferred temperature ranges. When benzene (b.p., 80°C.) is the aromatic compound of choice, the vapor phase nitration may be conducted at temperatures which encompass the entire operative temperature range, that is, from about 80°C. to about 300°C. Again, however, temperatures from about 150°C. to about 250°C. are preferred.

In a similar manner, when a solid compound such as naphthalene or benzoic acid (sublimation temperatures at atmospheric pressure, 80.2°C. and 100°C., respectively) is the aromatic compound of choice, the vapor phase nitration may be conducted at temperatures at or above the vaporization (sublimation) temperature, and preferably within the aforesaid preferred temperature range.

Notwithstanding the stated preferred temperature range, it will be appreciated that higher temperatures may be advantageously employed for more difficult to nitrate aromatic compounds. For example, o-dichlorobenzene (b.p., 179°C.) does not readily undergo nitration within the preferred temperature range of about 150°C. to about 250°C. Thus, in order to effect reasonable conversions and yields, temperatures greater than 250°C. to about 300°C. are preferred.

As previously indicated, the vapor phase nitration of this invention can be conducted at temperatures ranging from about 80°C. to about 300°C., with temperatures from about 150°C. to about 250°C. being preferred. Some advantages accruing from conducting the vapor phase nitration of this invention at the preferred temperatures include:

(a) greater selectivity to the desired nitro-aromatic compounds;
(b) little, if any, by-product formation (to contaminate the desired product);
(c) high material balance between reactants and products; and
(d) minimal thermal decomposition of the nitrogen dioxide.

The latter advantage [(d)] is particularly significant in that it, to a large extent, influences the remaining advantages. It, of course, is well-known in the art that at elevated temperatures nitrogen dioxide undergoes thermal decomposition into the inert (for purposes of this invention) nitric oxide and molecular oxygen. The decomposition begins at about 150°C. and is complete at about 620°C. The decomposition at various temperatures is as follows:

| | | | | | | |
|---|---|---|---|---|---|---|
| Temperature, °C. | 130 | 150 | 184 | 279 | 494 | 620 |
| Decomposition, % | 0 | 3 | 5 | 13 | 56.5 | 100 |

Thus, at temperatures between about 80°C. and about 300°C., the maximum loss of active nitrogen dioxide by thermal decomposition into inert nitric oxide is only about 15—20%, while at temperatures greater than 300°C., the loss by thermal decomposition rapidly increases to 30% or more, and, finally, to 100% at 620°C. Clearly, the magnitude of the loss of nitrogen dioxide at temperatures higher than the usual operating temperatures of this invention and, in particular, the preferred temperature ranges, is wasteful and impractical. Moreover, if recirculation of the effluent stream from such high temperature processes is desired, in order to prevent the complete loss of inert nitric oxide, it is necessary to employ an additional step to reoxidize the nitric oxide to the active nitrogen dioxide by treatment thereof with oxygen or an oxygen-containing gas such as air, with the attendant added cost and complexity. The additional cost and complexity of this added step, however, is substantially reduced or eliminated altogether by the usual operating temperature conditions employed in the process of this invention.

Pressure is not critical in the process of this invention. The vapor phase nitration reaction may be carried out at subatmospheric, atmospheric, or super-atmospheric pressures as desired. It will be appreciated that pressures in excess of atmospheric pressure may be advantageously employed as an aid in minimizing the previously discussed thermal decomposition of nitrogen dioxide, while subatmospheric pressures may be employed as an aid in vaporizing more difficult to vaporize aromatic compounds. It will be generally preferred, however, to conduct the reaction at or near atmospheric pressure. Generally, pressures from about $2.53 \times 10^4$ pascals or Pa (0.25 atmosphere or atm) to about $4.053 \times 10^5$ Pa (4.0 atm) may be conveniently employed.

The vapor phase nitration process of this invention is carried out in the presence of water, which is believed necessary to create and renew reaction sites on the nitration promoting catalyst. The required water can be supplied by water of hydration in the catalyst or, alternatively, by the separate addition of water via the feed stream. When water of hydration (within the previously defined substantially anhydrous limitation) is present, no added water is required since once the reaction is initiated, water produced during the course of the reaction (1 mole of water for each 2 moles of nitro-aromatic compound produced) is sufficient to sustain it.

If the nitration promoting catalyst is substantially free of water of hydration, it then becomes necessary to add water in an amount sufficient to provide the required reaction sites. Separate addition of water is usually preferred to ensure its presence in sufficient amount. The amount of water present, however, is not narrowly critical. Thus, amounts ranging from nominal or trace amounts (about 0.1 volume percent) up to about 15 percent by volume of the feed stream are generally sufficient, with amounts ranging from about 0.5 percent to about 5 percent by volume being desirably used.

The vapor phase nitration of this invention is conveniently carried out in an apparatus of the type suitable for carrying out chemical reactions in the vapor phase. It can be conducted in a single reactor or in multiple reactors using either a fixed bed, moving bed or a fluidized bed system to effect contacting of the reactants and the nitration promoting catalyst. Reaction is generally carried out by continuously passing a vaporous mixture of the aromatic compound and nitrogen dioxide over a bed of the nitration promoting catalyst while maintaining a temperature from about 80°C. to about 300°C., and, usually, about 175°C. to about 225°C.

The reactant aromatic compound can be pre-heated to form a vapor which is then admixed with gaseous nitrogen dioxide in a suitable reactor in predetermined relative proportions. The vaporous aromatic compound can be pumped into the reactor at a constant rate and admixed with a water-containing or humidified stream of gas and nitrogen dioxide before contacting the heated catalyst bed, or, alternatively, it can be conveniently swept into the reactor at a constant rate by a water-containing stream of carrier gas and thence admixed with a continuous stream of nitrogen dioxide before contacting the heated catalyst bed. The reactants can be charged into the reactor at any suitable flow rate.

As previously indicated, the reactant materials can be conveniently swept into the reactor by a stream

of carrier gas. The carrier gas employed in the present process can be oxygen or an oxygen-containing gas, for example, air, or an inert gas such as nitrogen, helium, and the like. When employed, it is advantageous to employ oxygen or an oxygen-containing gas as the carrier gas (for the aromatic compound) due to the stoichiometry of the nitration reaction between the aromatic compound and the nitrogen dioxide. In addition, carrier gases preferred for the required water and nitrogen dioxide, respectively, are air and nitrogen.

In the initial nitration reaction between the aromatic compound and the nitrogen dioxide, it is believed that for each 2 moles of aromatic compound, 3 moles of nitrogen dioxide are required to produce 2 moles of nitroaromatic compound, 1 mole of nitric oxide, and 1 mole of water. In the absence of an oxygen source such as supplied by the oxygen-containing carrier gas, the nitric oxide is lost, thereby reducing the nitrogen dioxide selectivity to the nitroaromatic compound by at least 33% (1/3), as well as the material balance between reactants and recovered products. In the presence of oxygen (and the nitration promoting catalyst), however, the nitric oxide undergoes the known reoxidation to nitrogen dioxide (stoichiometrically requiring 1 mole of oxygen for each 2 moles of nitric oxide), which undergoes further reaction with additional aromatic compound. This known reoxidation of nitric oxide to nitrogen dioxide also serves to reduce the loss of nitrogen dioxide as nitric oxide via the previously discussed nitrogen dioxide thermal decomposition. Overall, therefore, little, if any, nitrogen dioxide is lost by virtue of stoichiometrically produced, as well as thermally produced, nitric oxide.

The concentration of the aromatic compound in the feed mixture is not narrowly critical. All that is necessary is that the concentration be sufficient to permit the reaction to proceed at a reasonable rate. On the other hand, since the nitroaromatic compound produced will have a high vaporization temperature (for example, nitrochlorobenzene isomers, b.p., 235—246°C.), the concentration should be such that the nitroaromatic compound produced will not condense in the reactor. In addition, since mixtures of aromatic compounds and air (the preferred aromatic compound carrier gas) are potentially flammable and explosive, it is preferred, from a practical viewpoint, to operate at concentrations outside the flammable and explosive limits of the aromatic compound being employed. Generally, concentrations between about 1% and about 15% by volume are desirably employed.

The relative proportions of reactants generally can range from about 0.5 to 5 moles of nitrogen dioxide per mole of aromatic compound and, preferably, a ratio of about 1.5 to 4:1 is used.

The present process is suited to either batch or continuous operation. Continuous operations can involve recirculation of the effluent stream unreacted aromatic compound and nitrogen dioxide following isolation of the nitroaromatic compound product. Additional reactants—aromatic compounds and nitrogen dioxide—can then be charged to the reactor along with the recirculated stream to continue the process in a subsequent and continuous reaction. It will be noted that the substantial absence of side reactions, such as, for example, the thermal decomposition of nitrogen dioxide and undesired by-product formation advantageously facilitate such continuous operations in that extensive purification of the effluent stream is not required and, as previously noted, the cost and complexity of re-oxidation of the nitric oxide to nitrogen dioxide is substantially reduced or eliminated altogether.

The nitroaromatic compounds produced during the course of the vapor phase reaction can be collected in a suitable chilled container, and purified by any appropriate method and means known to the art such as, for example, distillation and crystallization. Fractional crystallization in accordance with conventional procedures are especially convenient for the separation of ortho and para isomers when a monosubstituted aromatic compound having an ortho-para orientation substituent, such as chlorobenzene, is employed as the reactant or starting material.

The recovered unreacted reactants, due to the substantial absence of side-reactions to produce undesirable by-products, are easily recycled to the reactor for further processing.

The following specific examples illustrating the best presently known method of practicing this invention are described in detail in order to facilitate a clear understanding of the invention. It should be understood, however, that the detailed expositions of the application of the invention while indicating preferred embodiments, are given by way of illustration only.

Examples 1—15

Preparation of catalysts

A stainless steel tube 40.64 cm (16 inches) in length and 2.54 cm (1 inch) outside diameter, was employed as the reactor. An alumina-silica-metal oxide combination material was placed in the reactor and dried, if necessary, by heating to about 225°C. under a constant stream of dry nitrogen for about 1 hour. The temperature was set at the preparation temperature, usually 175°C., and sulfur dioxide, along with nitrogen dioxide (in a nitrogen carrier stream), unless specified otherwise, was charged to the reactor containing the alumina-silica-metal oxide combination in approximately a 2—3/l mole ratio until the sulfur trioxide uptake had reached the desired amount. The time period was usually about 1 hour. The parameters and results are tabulated in Table 1.

TABLE 1
Alumina-silica-metal oxide combination[1]

| (Example) catalyst | Name[2] | $(Al_2O_3)_a(SiO_2)_b(M_{2/n}O)_c$ a | b Wt % | c |
|---|---|---|---|---|
| 1 | Boehmite[8] | 100 | 0 | 0 |
| 1a[9] | ″ | ″ | ″ | ″ |
| 2 | Zeolon 900H[8] | 9.4 | 90.6 | ″ |
| 2a[10] | ″ | ″ | ″ | ″ |
| 2b[11] | ″ | ″ | ″ | ″ |
| 3 | Silica-alumina[12] | 12 | 87 | ″ |
| 3a[13] | ″ | ″ | ″ | ″ |
| 3b[14] | ″ | ″ | ″ | ″ |
| 4 | ″ | ″ | ″ | ″ |
| 5[15] | Silica-alumina[16] | 50 | 50 | ″ |
| 5a[17] | ″ | ″ | ″ | ″ |
| 6 | Silica[18] | 0 | 100 | ″ |
| 7 | Silica gel[19] | ″ | ″ | ″ |
| 8[20] | Cobalt oxide-molybdenum oxide on alumina[16] | 86.5 | 0 | 13.5[21] |
| 8a[22] | ″ | ″ | ″ | ″ |
| 9[23] | ″ | 80.5 | ″ | 19.5[24] |
| 10[25] | Silica-alumina[26] | 45 | 53 | 0 |
| 10a[27] | ″ | ″ | ″ | ″ |
| 10b[28] | ″ | ″ | ″ | ″ |
| 10c[29] | ″ | ″ | ″ | ″ |
| 11[30] | Nickel oxide-tungsten oxide on silica-alumina[12] | 17.4 | 65 | 17.6[31] |
| 12[32] | Cobalt oxide on silica (α-quartz)[16] | 0 | 83.7 | 16.3[33] |
| 13[33] | Alumina[8] | 100 | 0 | 0 |
| 14 | Silica-alumina[12] | 12 | 87 | 0 |
| 15 | ″ | ″ | ″ | ″ |

TABLE 1 (Cont'd)
Alumina-silica-metal oxide combination[1]

| (Example) catalyst | Physical properties: Surface area $m^2/g$ | Physical properties: Form size, cm. | Shape | Amount, g: Initial[3] | Amount, g: Final[4] |
|---|---|---|---|---|---|
| 1 | 292.2 | 0.32×0.32 | Pellets | 112.9 | 103.6 |
| 1a[9] | " | " | " | " | " |
| 2 | 400—450 | " | " | 98.9 | 86.1 |
| 2a[10] | " | " | " | " | " |
| 2b[11] | " | " | " | " | " |
| 3 | 425—450 | " | " | 84.7 | 83.1 |
| 3a[13] | " | " | " | " | " |
| 3b[14] | " | " | " | " | " |
| 4 | " | " | " | 83.6 | 80.4 |
| 5[15] | 260 | 0.64×0.64 | Tablets | — | 105.5 |
| 5a[17] | " | " | " | " | " |
| 6 | 350 | 0.14—0.48 | Spheres | — | 54.8 |
| 7 | — | 0.12—0.34 | Crystallite | 92.4 | 90.4 |
| 8[20] | — | 0.32×0.32 | Pellets | — | 100.4 |
| 8a[22] | " | " | " | " | " |
| 9[23] | — | 0.34—0.48 | Spheres | — | 125.2 |
| 10[25] | " | 0.38×0.38 | Pellets | " | 120.1 |
| 10a[27] | " | " | " | " | " |
| 10b[28] | " | " | " | " | " |
| 10c[29] | " | " | " | " | " |
| 11[30] | 230 | 0.21×0.21 | Pellets | " | 101.0 |
| 12[32] | 110 | 0.40—0.64 | Spheres | " | " |
| 13[33] | 306 | 0.32 | " | " | 92.8 |
| 14 | 425—450 | 0.32×0.32 | Pellets | " | 87.0 |
| 15 | " | " | " | " | 91.3 |

TABLE 1 (Cont'd)

| | Sulfur trioxide uptake | | | Catalyst preparation conditions | | | | |
|---|---|---|---|---|---|---|---|---|
| | | | | flow rate ml/min. | | | | |
| (Example) catalyst | g, | wt. %[5] | (Totals)[6] | sulfur dioxide | nitrogen dioxide | carrier gas[7] | Time hours | Temp. °C. |
| 1 | 12.5, | 12.1 | | 107.0 | 49.0 | 31.0 | 1.0 | 175 |
| 1a[9] | 8.9, | 8.6 | (21.4, 20.6) | 129.0 | 154.0 | " | " | " |
| 2 | 9.1, | 10.6 | | 107.6 | 53.6 | " | " | " |
| 2a[10] | 5.2, | 6.0 | (14.3, 16.6) | 108.0 | 54.0 | " | " | " |
| 2b[11] | 1.1, | 1.3 | (15.4, 17.9) | " | " | " | " | " |
| 3 | 7.9, | 9.5 | | 107.0 | " | " | " | " |
| 3a | 11.8, | 14.2 | (19.7, 23.7) | " | 60.0 | " | " | " |
| 3b | 10.1, | 12.2 | (29.8, 35.9) | " | " | 26.3 | " | " |
| 4 | 32,2, | 40.0 | | " | 62.9 | 30.0 | 3.0 | 180 |
| 5[15] | 10.6, | 10.0 | | " | 48.8 | 31.0 | 1.0 | 175 |
| 5a[17] | 11.6, | 11.0 | (22.2, 21.0) | " | " | " | " | " |
| 6 | 11.5, | 21.0 | | " | " | " | 1.5 | " |
| 7 | 12.3, | 13.6 | | " | 60.0 | " | 1.0 | " |
| 8[20] | 5.8, | 5.8 | | 141.0 | 62.0 | " | 0.5 | " |
| 8a[22] | 4.7, | 4.7 | (10.5, 10.5) | 131.0 | 56.0 | " | 0.5 | " |
| 9[23] | 8.4, | 6.7 | | 131.0 | 46.0 | 31.0 | 1.0 | 175 |
| 10[25] | 6.1, | 5.1 | | 142.0 | 67.4 | " | 0.5 | " |
| 10a[27] | 2.7, | 2.2 | (8.8, 7.3) | 58.0 | 30.0 | " | " | " |
| 10b[28] | 2.9, | 2.4 | (11.7, 9.7) | 78.0 | 36.0 | " | " | " |
| 10c[29] | 4.8, | 4.0 | (16.5, 13.7) | 124.0 | 59.0 | " | " | " |
| 11[30] | 5.2, | 5.1 | | 128.0 | 41.0 | " | 1.0 | " |
| 12[32] | 12.5, | 14.6 | | 150.0 | 60.0 | " | " | " |
| 13[33] | 11.2, | 12.1 | | 53.5 | 54.0 | 21.3 | 2.0 | " |
| 14 | 19.6, | 22.5 | | 126.0 | 55.0 | 31.0 | 2.5 | " |
| 15 | 20.5, | 22.5 | | " | " | " | 2.2 | " |

[1] Composition and properties provided by commercial supplier unless otherwise noted.
[2] Material added initially to reactor.
[3] Weight in grams prior to drying, if subsequently dried.
[4] Weight in grams after drying, if dried.
[5] Based on the weight of the alumina-silica-metal oxide combination material.

[6] The total amount of sulfur trioxide taken up by the alumina-silica-metal oxide combination material as a result of a second, third, and the like addition, as applicable, of a sulfur trioxide source, usually sulfur dioxide and nitrogen dioxide, to a previously prepared adduct.

[7] Nitrogen; carrier gas for nitrogen dioxide.

[8] Available commercially from Norton Company, Akron, Ohio 44309.

[9] Catalyst 1, after use, was purged with steam at 225°C. for 1 hour, followed by dry nitrogen at 225°C. for an additional hour, and then contacted with additional sulfur dioxide and nitrogen dioxide to provide a total sulfur trioxide uptake.

[10] Catalyst 2, after two vapor phase nitration runs, was treated as described in Footnote 9.

[11] Catalyst 2a, after one vapor phase nitration run, was treated as described in Footnote 9.

[12] Available commercially from Strem Chemicals, Inc., Newburyport, Massachusetts 01950; contained 1.0% unidentified material.

[13] Catalyst 3, after two vapor phase nitration runs, was treated with additional sulfur dioxide and nitrogen dioxide for 1 hour.

[14] Catalyst 3a, after one vapor phase nitration run, was treated as described in Footnote 13.

[15] After one vapor phase nitration run, on virgin silica-alumina (catalyst precursor) for comparative purposes (Example 34), the catalyst composition of this invention was prepared.

[16] Available commercially from United Catalysts, Inc., Louisville, Kentucky 40232.

[17] Catalyst 5, after one vapor phase nitration run, was treated as described in Footnote 13.

[18] Available commercially from Air Products and Chemicals, Inc., Allentown, Pennsylvania 18105.

[19] Available commercially from Fisher Scientific Company, Fairhaven, New Jersey 07410.

[20] After one vapor phase nitration run on virgin alumina-silica-metal oxide combination material (catalyst precursor) for comparative purposes (Example 46), the catalyst composition of this invention was prepared.

[21] M represents a mixture of cobalt (n=2; 3.5%) and molybdenum (n=6; 10.0%) such that c is 13.5%.

[22] Catalyst 8, after one vapor phase nitration run, was treated with sulfur dioxide and nitrogen dioxide for an additional 30 minutes.

[23] After one vapor phase nitration run on virgin alumina-silica-metal oxide combination material (catalyst precursor) for comparative purposes (Example 49), the catalyst composition of this invention was prepared by treating the alumina-silica-metal oxide combination with sulfur dioxide and nitrogen dioxide for 1 hour.

[24] M represents a mixture of cobalt (n=2; 4.5%) and molybdenum (7=6; 15.0%) such that c is 19.5%.

[25] After one vapor phase nitration run on virgin alumina-silica-metal oxide combination material (catalyst precursor) for comparative purposes (Example 51), the catalyst composition of this invention was prepared.

[26] Available commercially from Ventron Corporation, Alfa Products, Danvers, Massachusetts 01923; contained 2.0% unidentified materials.

[27] Catalyst 10, after one vapor phase nitration run, was treated with additional sulfur dioxide and nitrogen dioxide.

[28] Catalyst 10a, after one vapor phase nitration run, was treated with additional sulfur dioxide and nitrogen dioxide.

[29] Catalyst 10b, after one vapor phase nitration run, was treated with additional sulfur dioxide and nitrogen dioxide.

[30] After one vapor phase nitration run on virgin alumina-silica-metal oxide combination material (catalyst precursor) for comparative purposes (Example 56), the catalyst composition was prepared.

[31] M represents a mixture of nickel (n=2; 3.3%) and tungsten (n=6; 14.3%) such that c is 17.6%. Composition determined by semiquantitative x-ray fluorescence.

[32] After one vapor phase nitration run on virgin alumina-silica-metal oxide combination material (catalyst precursor) for comparative purposes (Example 58), the catalyst composition was prepared.

[33] M represents cobalt (n=2 and 3; 16.3%).

Examples 16—74

Nitration reaction

Using the reactor system described in Examples 1—15 for preparation of the catalysts, a number of reactions were run to show the effectiveness of the nitration promotion catalyst compositions as catalysts in the vapor phase nitration of aromatic compounds.

A stream of aromatic compound was preheated and charged to the reactor tube in a humidified or water-containing stream of air. The nitrating agent, nitrogen dioxide unless otherwise specified, in a nitrogen carrier stream was mixed with the aromatic compound/air stream shortly before contact with the heated catalyst. The products were collected in a series of three chilled containers, the first of which was chilled in an ice water bath and the second and third of which were chilled in dry ice baths. Analyses were performed by gas chromatography on a Varian Associates Model 3700 instrument using a 1.83-meter (6-ft.) by 0.32-cm (0.125-inch) outside diameter column, packed with 0.5 percent phosphoric acid on 5/95 weight percent SP-1000/Chromosorb G [carboxylic acid terminated poly(ethylene nitroterephthalate) from

poly(ethylene glycol), M.W., 20,000, and nitroterephthalic acid, Supelco, Inc., Bellefonte, PA 16823/ diatomaceous earth, Johns-Manville Products Corp., Manville, NJ 08835] and programmed from 90°C. to 210°C. at a program rate of 10°C./min. The parameters and results are tabulated in Table 2.

TABLE 2

| | | Aromatic compound, R-$C_6H_5$ | | | | | |
|---|---|---|---|---|---|---|---|
| Example | Catalyst number | R | Flow rate ml/min. | g, moles | Conc. vol. % | Temp. °C. | Carrier gas[3] flow rate ml/min. |
| 16 | 1 | Cl | 22.38 | 27.0, 0.24 | 3.1 | 80 | 500.0 |
| 17 | 1a | " | 24.83 | 29.9, 0.27 | 3.4 | " | " |
| 18 | 2 | " | 27.40 | 41.3, 0.37 | 3.7 | " | " |
| 19 | 2a | " | 30.27 | 31.9, 0.28 | 4.2 | " | " |
| 20 | 2b | " | 12.03 | 14.8, 0.13 | 1.8 | " | " |
| 21 | 3 | " | 21.80 | 23.5, 0.21 | 3.0 | " | " |
| 22 | 3 | " | 25.83 | 46.7, 0.42 | 3.5 | " | " |
| 23 | 3a | " | 30.02 | 45.2, 0.40 | 4.1 | " | " |
| 24 | 3b | " | 17.86 | 32.3, 0.29 | 2.5 | " | " |
| 25 | 4 | " | 21.88 | 36.3, 0.32 | 3.1 | 85 | " |
| 26 | 4 | " | 21.89 | 39.6, 0.35 | 3.2 | " | " |
| 27 | 4[6] | " | 20.79 | 31.3, 0.28 | 3.0 | " | " |
| 28 | 4[7] | " | 69.96 | 116.0, 1.03 | 6.4 | " | 850.0 |
| 29 | 4[7] | " | 40.02 | 72.4, 0.64 | 5.0 | " | 600.0 |
| 30 | 4[7] | " | 25.27 | 45.7, 0.41 | 3.6 | " | 500.0 |
| 31 | 4[8] | " | 23.71 | 35.7, 0.32 | 3.3 | " | " |
| 32 | 4[9] | H | 93.26 | 48.7, 0.62 | 10.7 | 30 | " |
| 33 | 4[10] | H | 87.97 | 36.8, 0.47 | 9.8 | " | " |
| 34 | 5-P[5] | Cl | 31.47 | 66.8, 0.59 | 4.3 | 85 | 500.0 |
| 35 | 5 | " | 31.73 | 56.9, 0.51 | 4.4 | " | " |
| 36 | 5a | Cl | 28.00 | 51.1, 0.45 | 3.8 | 85 | 500.0 |
| 37 | 5a | " | 14.93 | 31.6, 0.28 | 2.1 | " | " |
| 38 | 6 | " | 31.24 | 47.1, 0.42 | 4.3 | " | " |
| 39 | 6 | " | 29.05 | 43.8, 0.39 | 4.0 | " | " |
| 40 | 6 | " | 17.42 | 31.0, 0.28 | 2.5 | " | " |
| 41 | 7 | " | 21.92 | 33.0, 0.29 | 3.0 | 80 | " |
| 42 | 7 | " | 30.23 | 54.7, 0.49 | 4.1 | " | " |

TABLE 2 (Cont'd)

| Example | Catalyst Number | Aromatic compound, R-$C_6H_5$ R | Flow rate ml/min. | g, moles | Conc. vol. % | Temp. °C. | Carrier gas[3] flow rate ml/min. |
|---|---|---|---|---|---|---|---|
| 43 | 7 | Cl | 24.87 | 52.5, 0.47 | 3.5 | 80 | 500.0 |
| 44 | 7 | " | 19.22 | 34.8, 0.31 | 2.8 | " | " |
| 45 | 7 | " | 23.80 | 43.0, 0.38 | 3.5 | " | " |
| 46 | 8-P[5] | " | 25.79 | 42.1, 0.38 | 3.3 | 82 | " |
| 47 | 8 | " | 24.64 | 36.9, 0.33 | 3.2 | " | " |
| 48 | 8a | " | 24.12 | 47.6, 0.42 | 3.2 | " | " |
| 49 | 9-P[5] | " | 21.89 | 38.5, 0.34 | 2.9 | " | " |
| 50 | 9 | " | 13.44 | 20.2, 0.18 | 2.7 | " | 320.0 |
| 51 | 10-P[5] | " | 23.15 | 34.7, 0.31 | 3.2 | 85 | 500.0 |
| 52 | 10 | " | 22.23 | 31.4, 0.28 | 2.9 | 82 | " |
| 53 | 10a | " | 23.02 | 41.6, 0.37 | 3.0 | " | " |
| 54 | 10b | " | 23.02 | 41.5, 0.37 | 3.0 | " | " |
| 55 | 10c | " | 21.72 | 35.8, 0.32 | 2.9 | " | " |
| 56 | 11-P[5] | " | 23.02 | 41.2, 0.37 | 3.0 | " | " |
| 57 | 11 | " | 12.69 | 18.6, 0.17 | 2.5 | " | 320.0 |
| 58 | 12-P[5] | " | 21.04 | 35.3, 0.31 | 2.7 | " | 500.0 |
| 59 | 12 | " | 23.15 | 35.1, 0.31 | 3.0 | " | " |
| 60 | 13-P[5] | " | 22.44 | 23.1, 0.21 | 3.1 | 80 | " |
| 61 | 13 | " | 19.98 | 27.1, 0.24 | 2.8 | " | " |
| 62 | 14[11] | H | 54.06 | 56.5, 0.72 | 9.4 | 21 | — |
| 63[12] | " | " | 54.60 | 57.0, 0.73 | 8.6 | " | — |
| 64 | " | " | 42.71 | 44.6, 0.57 | 7.5 | 21 | — |
| 65[12] | " | " | 40.92 | 42.8, 0.55 | 6.9 | " | — |
| 66 | " | " | 41.88 | 43.8, 0.56 | 7.3 | 23 | — |
| 67 | " | " | 40.27 | 42.1, 0.54 | 7.3 | 22 | — |
| 68 | " | " | 40.99 | 47.1, 0.60 | 7.2 | 18 | — |
| 69 | " | $CH_3$ | 30.99 | 35.2, 0.38 | 5.9 | 21 | — |
| 70 | " | " | 29.09 | 41.3, 0.45 | 5.6 | " | — |
| 71 | " | " | 33.45 | 41.2, 0.45 | 4.7 | " | — |

TABLE 2 (Cont'd)

| Example | Catalyst number | Aromatic compound, R-$C_6H_5$ R | Flow rate ml/min. | g, moles | Conc. vol. % | Temp. °C. | Carrier gas[3] flow rate ml/min. |
|---|---|---|---|---|---|---|---|
| 72 | 14[11] | $CH_3$ | 36.50 | 40.1, 0.44 | 5.0 | 21 | — |
| 73 | 15[13] | $C_2H_5O$ | 38.35 | 58.6, 0.55 | 6.7 | " | — |
| 74 | " | " | 27.38 | 44.8, 0.42 | 2.5 | " | — |

TABLE 2 (Cont'd)

| Example | Nitrating agent[1] Flow rate ml/min. | g, moles | Conc. vol. % | Temp. °C. | Carrier gas[4] flow rate ml/min. | Nitrating agent/-aromatic compound molar ratio |
|---|---|---|---|---|---|---|
| 16 | 54.66 | 26.9, 0.58 | 7.7 | 15 | 31.0 | 2.42 |
| 17 | 66.23 | 32.6, 0.71 | 9.1 | " | " | 2.63 |
| 18 | 56.34 | 34.7, 0.75 | 7.8 | " | " | 2.03 |
| 19 | 61.66 | 26.6, 0.59 | 8.5 | " | " | 2.11 |
| 20 | 41.44 | 20.8, 0.45 | 6.0 | " | " | 3.46 |
| 21 | 54.29 | 24.0, 0.52 | 7.6 | " | " | 2.48 |
| 22 | 65.02 | 48.1, 1.05 | 8.8 | " | " | 2.50 |
| 23 | 60.18 | 37.1, 0.81 | 8.2 | " | " | 2.02 |
| 24 | 45.11 | 33.4, 0.73 | 6.4 | " | " | 2.52 |
| 25 | 62.92 | 42.6, 0.93 | 8.8 | " | 30.0 | 2.91 |
| 26 | 38.82 | 28.7, 0.62 | 5.7 | " | 21.0 | 1.77 |
| 27 | 26.59 | 16.4, 0.36 | 3.9 | " | 19.0 | 1.29 |
| 28 | 39.91 | 27.0, 0.59 | 3.6 | " | " | 0.57 |
| 29 | 30.43 | 22.5, 0.49 | 3.8 | " | " | 0.77 |
| 30 | 33.78 | 25.0, 0.54 | 4.8 | " | " | 1.32 |
| 31 | 41.75 | 25.7, 0.56 | 5.8 | " | 21.0 | 1.75 |
| 32 | 98.69 | 30.4, 0.66 | 11.3 | " | 47.5 | 1.06 |
| 33 | 116.34 | 28.7, 0.62 | 13.0 | " | 69.0 | 1.32 |
| 34 | 49.07 | 42.1, 0.92 | 6.8 | " | 31.0 | 1.56 |
| 35 | 49.15 | 36.3, 0.79 | 6.8 | " | " | 1.55 |
| 36 | 53.51 | 39.4, 0.86 | 7.4 | " | " | 1.91 |
| 37 | 36.27 | 31.5, 0.68 | 5.1 | " | " | 2.43 |

TABLE 2 (Cont'd)

| Example | Nitrating agent[1] Flow rate ml/min. | g, moles | Conc. vol. % | Temp. °C. | Carrier gas[4] flow rate ml/min. | Nitrating agent/-aromatic compound molar ratio |
|---|---|---|---|---|---|---|
| 38 | 55.68 | 34.3, 0.75 | 7.6 | 15 | 31.0 | 1.79 |
| 39 | 51.13 | 31.5, 0.68 | 7.1 | " | " | 1.74 |
| 40 | 35.47 | 26.0, 0.57 | 5.1 | " | " | 2.04 |
| 41 | 60.63 | 37.4, 0.81 | 8.3 | " | " | 2.79 |
| 42 | 55.62 | 41.1, 0.89 | 7.6 | " | " | 1.82 |
| 43 | 48.00 | 41.4, 0.90 | 6.7 | " | " | 1.91 |
| 44 | 37.60 | 27.8, 0.60 | 5.5 | " | 21.0 | 1.94 |
| 45 | 37.01 | 27.4, 0.60 | 5.4 | " | " | 1.58 |
| 46 | 55.66 | 37.8, 0.82 | 7.2 | 12 | 31.0 | 2.16 |
| 47 | 53.76 | 33.2, 0.72 | 7.0 | " | " | 2.18 |
| 48 | 51.12 | 40.8, 0.89 | 6.7 | " | " | 2.12 |
| 49 | 48.92 | 34.8, 0.76 | 6.5 | " | " | 2.24 |
| 50 | 29.12 | 17.9, 0.39 | 5.7 | " | 24.0 | 2.17 |
| 51 | 57.49 | 35.6, 0.77 | 8.0 | " | 31.0 | 2.48 |
| 52 | 59.73 | 33.0, 0.72 | 7.8 | " | " | 2.57 |
| 53 | 52.89 | 39.3, 0.85 | 7.1 | " | " | 2.30 |
| 54 | 51.02 | 37.9, 0.82 | 6.7 | " | " | 2.22 |
| 55 | 52.27 | 35.6, 0.77 | 6.9 | " | " | 2.41 |
| 56 | 47.91 | 35.4, 0.77 | 6.3 | " | " | 2.08 |
| 57 | 25.39 | 15.8, 0.34 | 5.0 | " | 24.0 | 2.00 |
| 58 | 60.41 | 40.8, 0.89 | 7.8 | " | 31.0 | 2.87 |
| 59 | 52.27 | 32.3, 0.70 | 6.8 | " | " | 2.26 |
| 60 | 72.12 | 30.4, 0.66 | 9.9 | 15 | 30.0 | 3.14 |
| 61 | 69.80 | 38.7, 0.84 | 9.6 | " | " | 3.50 |
| 62 | 67.52 | 41.6, 0.90 | 11.7 | 10 | 29.0 | 1.25 |
| 63[12] | 97.42 | 60.0, 1.30 | 15.4 | 15 | 50.0 | 1.78 |
| 64 | 67.18 | 41.4, 0.90 | 11.8 | 10 | 29.0 | 1.58 |
| 65[12] | 85.07 | 52.4, 1.14 | 14.3 | 15 | 45.0 | 2.07 |
| 66 | 73.71 | 45.4, 0.99 | 12.9 | 10 | 29.0 | 1.77 |

TABLE 2 (Cont'd)

| Example | Nitrating agent[1] Flow rate ml/min. | g, moles | Conc. vol. % | Temp. °C. | Carrier gas[4] flow rate ml/min. | Nitrating agent/-aromatic compound molar ratio |
|---|---|---|---|---|---|---|
| 67 | 42.97 | 26.5, 0.58 | 7.8 | 10 | 29.0 | 1.07 |
| 68 | 69.35 | 47.0, 1.02 | 12.2 | " | " | 1.70 |
| 69 | 48.61 | 24.0, 0.52 | 9.2 | " | " | 1.37 |
| 70 | 37.22 | 22.9, 0.50 | 7.2 | " | " | 1.11 |
| 71 | 43.58 | 26.8, 0.58 | 6.1 | " | 36.5 | 1.29 |
| 72 | 62.12 | 38.3, 0.83 | 8.5 | 15 | " | 1.89 |
| 73 | 65.98 | 37.9, 0.82 | 11.6 | 10 | " | 1.49 |
| 74 | 68.29 | 42.1, 0.92 | 6.1 | " | " | 2.19 |

TABLE 2 (Cont'd)

| Example | Water Flow rate ml/min. | g, moles | Conc. vol. % | Temp. °C. | Carrier gas[3] flow rate ml/min. | Reaction conditions Temp. °C. | Time hours | Conversion, %[2] |
|---|---|---|---|---|---|---|---|---|
| 16 | 7.67 | 1.5, 0.082 | 1.1 | 80 | 98.0 | 175 | 4.0 | 41.0 |
| 17 | 6.84 | 1.3, 0.072 | 0.9 | " | " | " | " | 49.8 |
| 18 | 10.62 | 2.6, 0.14 | 1.5 | " | " | " | 5.0 | 77.6 |
| 19 | 7.70 | 1.3, 0.072 | 1.0 | " | " | " | 3.5 | 81.1 |
| 20 | 4.77 | 0.9, 0.050 | 0.7 | " | " | " | 4.1 | 96.4 |
| 21 | 12.33 | 2.1, 0.12 | 1.7 | " | " | " | 3.6 | 88.5 |
| 22 | 20.12 | 5.8, 0.32 | 2.7 | " | " | " | 6.0 | 66.4 |
| 23 | 16.55 | 4.0, 0.22 | 2.2 | " | " | " | 5.0 | 85.0 |
| 24 | 15.94 | 4.6, 0.26 | 2.3 | " | " | " | 6.0 | 93.4 |
| 25 | 3.13 | 0.8, 0.044 | 0.4 | 85 | " | 180 | 5.5 | 99.7 |
| 26 | 0.14 | 0.04, 0.0022 | 0.02 | " | " | " | 6.0 | 78.4 |
| 27 | 24.31 | 5.9, 0.33 | 3.5 | " | " | " | 5.0 | 67.7 |
| 28 | 17.61 | 4.7, 0.26 | 1.6 | " | " | " | 5.5 | 22.1 |
| 29 | 18.29 | 5.3, 0.29 | 2.3 | " | " | " | 6.0 | 36.1 |
| 30 | 27.86 | 8.1, 0.45 | 4.0 | " | " | " | " | 71.9 |
| 31 | 34.80 | 8.4, 0.47 | 4.8 | " | " | " | 5.0 | 88.1 |
| 32 | 38.00 | 4.6, 0.26 | 4.3 | " | " | 160 | 2.5 | 66.8 |

TABLE 2 (Cont'd)

| Example | Water: Flow rate ml/min. | Water: g, moles | Water: Conc. vol. % | Water: Temp. °C. | Water: Carrier gas[3] flow rate ml/min. | Reaction conditions: Temp. °C. | Reaction conditions: Time hours | Conversion, %[2] |
|---|---|---|---|---|---|---|---|---|
| 33 | 23.02 | 2.2, 0.12 | 2.6 | 85 | 98.0 | 160 | 2.0 | 83.1 |
| 34 | 16.53 | 5.6, 0.31 | 2.3 | " | " | 175 | 7.0 | 1.8 |
| 35 | 16.80 | 4.8, 0.27 | 2.3 | " | " | " | 6.0 | 49.8 |
| 36 | 16.80 | 4.9, 0.27 | 2.3 | " | " | " | 6.0 | 44.3 |
| 37 | 28.26 | 9.5, 0.53 | 4.0 | " | " | " | 7.0 | 51.7 |
| 38 | 12.86 | 3.1, 0.17 | 1.8 | " | " | " | 5.0 | 67.1 |
| 39 | 15.76 | 3.8, 0.21 | 2.2 | " | " | " | " | 74.2 |
| 40 | 14.93 | 4.3, 0.24 | 2.1 | " | " | " | 6.0 | 93.6 |
| 41 | 21.16 | 5.1, 0.28 | 2.9 | 80 | " | " | 5.0 | 90.9 |
| 42 | 15.73 | 11.6, 0.64 | 2.2 | " | " | " | 6.0 | 77.7 |
| 43 | 11.85 | 4.0, 0.22 | 1.7 | " | " | 180 | 7.0 | 86.9 |
| 44 | 7.81 | 2.3, 0.13 | 1.1 | " | " | 190 | 6.0 | 86.5 |
| 45 | 6.26 | 1.8, 0.10 | 0.9 | " | " | 185 | " | 70.2 |
| 46 | 25.12 | 6.7, 0.37 | 3.3 | 88 | 135.0 | 175 | 5.5 | 5.3 |
| 47 | 26.13 | 6.3, 0.35 | 3.4 | " | " | " | 5.0 | 52.4 |
| 48 | 22.97 | 7.2, 0.40 | 3.0 | " | " | " | 6.5 | 60.1 |
| 49 | 14.80 | 4.2, 0.23 | 2.0 | " | " | " | 5.8 | 3.0 |
| 50 | 16.43 | 4.0, 0.22 | 3.2 | " | 105.0 | " | 5.0 | 77.0 |
| 51 | 8.21 | 2.0, 0.11 | 1.1 | 85 | 98.0 | " | " | 35.6 |
| 52 | 19.91 | 4.3, 0.24 | 2.6 | 88 | 135.0 | " | 4.5 | 93.3 |
| 53 | 20.53 | 5.9, 0.33 | 2.7 | " | " | " | 6.0 | 77.2 |
| 54 | 24.27 | 7.0, 0.39 | 3.2 | " | " | " | " | 73.7 |
| 55 | 20.36 | 5.4, 0.30 | 2.7 | " | " | " | 5.5 | 81.9 |
| 56 | 23.02 | 6.6, 0.37 | 3.0 | " | " | " | 6.0 | 44.8 |
| 57 | 16.43 | 4.0, 0.22 | 3.3 | " | 105.0 | " | 5.0 | 88.8 |
| 58 | 22.40 | 6.0, 0.33 | 2.9 | " | 135.0 | " | 5.5 | 8.3 |
| 59 | 21.65 | 5.3, 0.29 | 2.8 | " | " | " | 5.0 | 94.3 |
| 60 | 8.89 | 1.5, 0.083 | 1.2 | 80 | 98.0 | " | " | 4.3 |

TABLE 2 (Cont'd)

| Example | Water: Flow rate ml/min. | Water: g, moles | Water: Conc. vol. % | Water: Temp. °C. | Carrier gas[3] flow rate ml/min. | Reaction conditions: Temp. °C. | Reaction conditions: Time hours | Conversion, %[2] |
|---|---|---|---|---|---|---|---|---|
| 61 | 6.91 | 1.5, 0.083 | 1.0 | 80 | 98.0 | 175 | 4.5 | 63.1 |
| 62 | 20.48 | 5.0, 0.28 | 3.6 | 40 | 405 | 155 | 5.0 | 65.9 |
| 63[12] | 25.80 | 6.2, 0.34 | 4.1 | 50 | " | " | " | 99.1 |
| 64 | 23.85 | 5.8, 0.32 | 4.2 | 40 | " | 154 | " | 84.6 |
| 65[12] | 19.41 | 4.7, 0.26 | 3.3 | " | " | 155 | " | 99.4 |
| 66 | 23.89 | 5.8, 0.32 | 4.2 | " | " | " | " | 90.0 |
| 67 | 30.95 | 7.5, 0.42 | 5.6 | 50 | " | 154 | " | 61.9 |
| 68 | 23.57 | 6.2, 0.34 | 4.2 | 40 | " | 134 | 5.5 | 78.7 |
| 69 | 14.31 | 2.8, 0.16 | 2.7 | 41 | " | 174 | 4.0 | 24.8 |
| 70 | 19.08 | 4.6, 0.26 | 3.7 | " | " | 154 | 5.0 | 11.5 |
| 71 | 23.44 | 5.6, 0.31 | 3.3 | " | 580 | 177 | " | 36.4 |
| 72 | 17.92 | 4.3, 0.24 | 2.4 | 40 | " | 172 | " | 48.7 |
| 73 | 23.91 | 5.4, 0.30 | 4.2 | " | 405 | 232 | 4.7 | 12.7 |
| 74 | 42.23 | 10.2, 0.57 | 3.8 | 34.5 | 940 | 192 | 5.0 | 27.6 |

TABLE 2 (Cont'd)

| Example | Products, %: R-$C_6H_4$-$NO_2$: R=H | R=$CH_3$, ortho | $C_2H_5O$, meta | Cl para | Unidentified by- | para/ ortho | Material balance: g In | Material balance: g Out | Material balance: % |
|---|---|---|---|---|---|---|---|---|---|
| 16 | — | 9.8 | 0.9 | 30.3 | — | 3.09 | 55.4 | 46.4 | 83.8 |
| 17 | — | 13.8 | 1.0 | 34.4 | — | 2.49 | 63.8 | 62.8 | 98.4 |
| 18 | — | 20.3 | 1.6 | 55.5 | 0.2 | 2.73 | 78.6 | 68.6 | 87.3 |
| 19 | — | 22.2 | 1.2 | 57.7 | — | 2.60 | 59.8 | 57.2 | 95.7 |
| 20 | — | 24.6 | 1.4 | 70.4 | — | 2.86 | 36.5 | 31.0 | 84.9 |
| 21 | — | 23.2 | 1.4 | 63.9 | <0.1 | 2.75 | 49.6 | 45.0 | 90.7 |
| 22 | — | 16.7 | 1.1 | 48.6 | <0.1 | 2.91 | 100.6 | 97.2 | 96.6 |
| 23 | — | 24.3 | 1.7 | 59.0 | — | 2.43 | 86.3 | 81.6 | 94.6 |
| 24 | — | 28.1 | 1.8 | 63.6 | — | 2.26 | 70.3 | 70.6 | 100.4 |

TABLE 2 (Cont'd)

| | Products, % | | | | | | Material balance | | |
|---|---|---|---|---|---|---|---|---|---|
| | $R-C_6H_4-NO_2$ | | | | | | g | | |
| Example | R=H | $R=CH_3$, ortho | $C_2H_5O$, meta | Cl para | Unidentified by- | para/ ortho | In | Out | % |
| 25 | — | 28.6 | 1.6 | 69.5 | <0.1 | 2.43 | 79.7 | 74.4 | 93.4 |
| 26 | — | 22.9 | 1.3 | 53.5 | 0.8 | 2.34 | 68.3 | 58.3 | 85.4 |
| 27 | — | 20.1 | 0.8 | 46.4 | 0.4 | 2.31 | 53.6 | 44.2 | 82.5 |
| 28 | — | 6.4 | 0.4 | 15.0 | 0.4 | 2.34 | 147.7 | 136.3 | 92.3 |
| 29 | — | 10.5 | 0.5 | 24.8 | 0.4 | 2.36 | 100.2 | 90.9 | 90.7 |
| 30 | — | 19.6 | 1.0 | 51.2 | 0.2 | 2.61 | 78.8 | 75.0 | 95.2 |
| 31 | — | 26.2 | 1.3 | 60.6 | 0.1 | 2.31 | 69.8 | 68.7 | 98.4 |
| 32 | 66.3 | — | — | — | 0.5 | — | 83.7 | 79.1 | 94.5 |
| 33 | 82.2 | — | — | — | 0.9 | — | 67.7 | 66.7 | 98.5 |
| 34 | — | 0.6 | — | 1.2 | — | 2.00 | 114.5 | 114.8 | 100.3 |
| 35 | — | 13.3 | — | 36.5 | — | 2.74 | 98.0 | — | — |
| 36 | — | 13.3 | — | 31.1 | — | 2.34 | 95.4 | 95.9 | 100.5 |
| 37 | — | 14.8 | 0.6 | 35.1 | — | 2.37 | 72.6 | 67.5 | 93.0 |
| 38 | — | 21.6 | 1.4 | 44.2 | — | 2.05 | 84.5 | 64.4 | 76.2 |
| 39 | — | 25.8 | 2.4 | 46.0 | — | 1.78 | 79.1 | 75.0 | 94.8 |
| 40 | — | 30.2 | 1.9 | 61.4 | — | 2.03 | 61.3 | 57.9 | 94.5 |
| 41 | — | 24.1 | 1.6 | 65.2 | — | 2.71 | 75.5 | 56.9 | 75.4 |
| 42 | — | 24.0 | 1.2 | 52.5 | — | 2.19 | 107.4 | 96.4 | 89.8 |
| 43 | — | 25.8 | 1.4 | 59.6 | — | 2.31 | 97.9 | 86.3 | 88.2 |
| 44 | — | 24.1 | 1.3 | 61.0 | — | 2.53 | 64.9 | 50.1 | 77.2 |
| 45 | — | 20.0 | 1.1 | 49.2 | — | 2.46 | 72.2 | 71.1 | 98.5 |
| 46 | — | 11.4 | 0.2 | 3.7 | — | 2.64 | 86.9 | 87.3 | 100.5 |
| 47 | — | 12.7 | 0.3 | 39.3 | — | 3.09 | 76.4 | 67.4 | 88.2 |
| 48 | — | 14.6 | 0.1 | 45.3 | — | 3.10 | 95.6 | 87.9 | 91.9 |
| 49 | — | 0.8 | 0.1 | 2.0 | — | 2.50 | 77.5 | 79.4 | 102.5 |
| 50 | — | 17.8 | 0.1 | 49.0 | — | 2.75 | 42.1 | 38.3 | 91.0 |
| 51 | — | 10.4 | 0.3 | 24.9 | — | 2.39 | 72.3 | 69.6 | 96.3 |
| 52 | — | 18.9 | 1.1 | 63.4 | — | 3.35 | 68.7 | 69.0 | 100.4 |

TABLE 2 (Cont'd)

| | Products, % | | | | | | Material balance | | |
|---|---|---|---|---|---|---|---|---|---|
| | R-$C_6H_4$-$NO_2$ | | | | | | g | | |
| Example | R=H | R=$CH_3$, ortho | $C_2H_5O$, meta | Cl para | Unidentified by- | para/ ortho | In | Out | % |
| 53 | — | 16.8 | 1.1 | 59.3 | — | 3.53 | 86.8 | 86.8 | 100.0 |
| 54 | — | 16.3 | 0.9 | 56.5 | — | 3.47 | 86.4 | 88.0 | 101.9 |
| 55 | — | 18.2 | 1.1 | 62.7 | — | 3.45 | 76.8 | 78.0 | 101.6 |
| 56 | — | 12.6 | 0.2 | 32.0 | — | 2.54 | 83.2 | 76.6 | 92.1 |
| 57 | — | 26.7 | 1.0 | 61.1 | — | 2.29 | 38.4 | 35.9 | 93.5 |
| 58 | — | 2.3 | 0.1 | 5.8 | — | 2.52 | 82.1 | 83.1 | 101.2 |
| 59 | — | 26.8 | 0.7 | 66.7 | <0.1 | 2.49 | 72.7 | 69.7 | 95.9 |
| 60 | — | 0.4 | 0.2 | 0.8 | 3.0 | 2.00 | 55.0 | 52.6 | 95.6 |
| 61 | — | 14.6 | 1.0 | 47.6 | — | 3.26 | 67.3 | 64.1 | 95.2 |
| 62 | 65.9 | — | — | — | — | — | 103.1 | 101.6 | 98.5 |
| 63[12] | 99.1 | — | — | — | — | — | 123.2 | 117.0 | 95.0 |
| 64 | 84.6 | — | — | — | — | — | 91.8 | 91.4 | 99.6 |
| 65[12] | 99.4 | — | — | — | — | — | 99.9 | 95.1 | 95.2 |
| 66 | 90.4 | — | — | — | — | — | 95.0 | 93.4 | 98.3 |
| 67 | 61.9 | — | — | — | — | — | 76.1 | 70.3 | 92.4 |
| 68 | 78.7 | — | — | — | — | — | 100.3 | 98.8 | 98.5 |
| 69 | — | 13.7 | — | 11.1 | — | 0.81 | 62.0 | 56.5 | 91.1 |
| 70 | — | 6.0 | — | 5.5 | — | 0.92 | 68.8 | 59.4 | 86.3 |
| 71 | — | 17.2 | — | 19.2 | — | 1.12 | 73.6 | 70.5 | 95.8 |
| 72 | — | 23.3 | — | 25.2 | 0.2 | 1.09 | 82.7 | 72.4 | 87.5 |
| 73 | — | 1.5 | 0.1 | 11.1 | — | 7.40 | 101.9 | 69.9 | 68.6 |
| 74 | — | 6.3 | — | 21.3 | — | 3.38 | 97.1 | 75.4 | 77.7 |

[1] Nitrogen dioxide (M.W., 46) unless specified otherwise.
[2] Based on the aromatic compound.
[3] Air
[4] Nitrogen
-p[5] Numbered catalyst precursor prior to treatment for sulfur trioxide uptake. Comparative run to demonstrate the effectiveness of the present invention over prior art catalysts.
[6] Pretreated catalyst for 15 minutes with nitrogen dioxide at operating conditions (for the vapor phase nitration in the absence of the aromatic compound).
[7] Pretreated catalyst as described in Footnote 6 for 12 minutes.
[8] Pretreated catalyst as described in Footnote 6 for 10 minutes.
[9] Pretreated catalyst as described in Footnote 6 for 3 minutes.

[10] No pretreatment.
[11] Catalyst 14 was employed as the catalyst in Examples 62—72 without further treatment with sulfur dioxide and nitrogen dioxide.
[12] Reaction was run at a gauge pressure of $1.03\times10^5$ pascal (Pa; 15 psig).
[13] Catalyst 15 was employed as the catalyst in Examples 73—74 without further treatment with sulfur dioxide and nitrogen dioxide.

Examples 75—78

The following examples were run to illustrate the use of the nitration promotion catalyst compositions in the vapor phase nitration of disubstituted aromatic compounds using o- or 1,2- dichlorobenzene as a typical compound.

The reactor system described in Examples 1—15 and the procedure described in Examples 16—74 were employed. The parameters and results are tabulated in Table 3.

TABLE 3

| Example | Catalyst number | Aromatic compound, $R—C_6H_4—R^1$: R | $R^1$ | Flow rate ml/min. | g, moles | Conc. vol. % | Temp. °C. | Carrier gas[3] flow rate ml/min. |
|---|---|---|---|---|---|---|---|---|
| 75 | 14[5] | 1-Cl | 2-Cl | 46.11 | 90.8, 0.62 | 8.1 | 21 | — |
| 76 | 14 | " | " | 40.51 | 79.7, 0.54 | 7.4 | " | — |
| 77 | 14 | " | " | 43.48 | 85.6, 0.58 | 7.1 | " | — |
| 78 | 14 | " | " | 43.26 | 85.2, 0.58 | 5.6 | " | — |

TABLE 3 (Cont'd)

| Example | Nitrating agent[1]: Flow rate ml/min. | g, moles | Conc. vol. % | Temp. °C. | Carrier gas[4] flow rate ml/min. | Nitrating agent/-aromatic compound molar ratio |
|---|---|---|---|---|---|---|
| 75 | 64.57 | 39.8, 0.87 | 11.3 | 10 | 36.5 | 1.40 |
| 76 | 49.23 | 30.3, 0.66 | 9.0 | " | " | 1.22 |
| 77 | 102.42 | 63.1, 1.37 | 16.6 | " | 45.0 | 2.36 |
| 78 | 99.21 | 61.1, 1.33 | 12.5 | " | " | 2.29 |

TABLE 3 (Cont'd)

| Example | Water: Flow rate ml/min. | g, moles | Conc. vol. % | Temp. °C. | Carrier gas[3] flow rate ml/min. | Reaction conditions: Temp. °C. | Time hours | Conversion, %[2] |
|---|---|---|---|---|---|---|---|---|
| 75 | 20.16 | 4.9, 0.127 | 3.5 | 41 | 405 | 175 | 5.0 | 1.1 |
| 76 | 17.17 | 4.1, 0.23 | 3.1 | 40 | " | 206 | " | 3.0 |
| 77 | 20.78 | 5.0, 0.28 | 3.4 | " | " | 255 | " | 16.2 |
| 78 | 33.27 | 8.0, 0.44 | 4.2 | " | 570 | 281 | " | 29.3 |

TABLE 3 (Cont'd)

| | Products, % | | | | | | |
|---|---|---|---|---|---|---|---|
| | $R^1$ \| R—$C_6H_3$—$NO_2$ | | | | Material balance | | |
| | $R=R^1=Cl$ | | | | g | | |
| Example | 2,3- | 3,4- | By- | 3,4/2,3- | In | Out | % |
| 75 | 0.05 | 0.5 | 0.55 | 10.00 | 135.5 | 123.9 | 91.4 |
| 76 | 0.4 | 2.0 | 0.6 | 5.00 | 114.1 | 110.7 | 97.0 |
| 77 | 2.6 | 11.8 | 1.8 | 4.54 | 153.7 | 146.1 | 95.1 |
| 78 | 3.5 | 19.6 | 6.2 | 5.60 | 154.3 | 148.6 | 96.3 |

[1] Nitrogen dioxide (M.W., 46) unless specified otherwise.
[2] Based on the aromatic compound.
[3] Air
[4] Nitrogen
[5] Catalyst 14 after being employed as the catalyst in Examples 62—72 above, was employed in Examples 75—78 without further treatment with sulfur dioxide and nitrogen dioxide.

**Claims**

1. A catalyst composition comprising the adduct of:
(a) an alumina-silica-metal oxide combination represented by the formula

$$(Al_2O_3)_a(SiO_2)_b(M_{2/n}O)_c$$

wherein M is a metal cation selected from the lanthanides, Groups 1b, 2b, 5b, 6b, 7b, and 8 of the Periodic Table of the Elements, and mixtures thereof, and a, b, and c represent weight percent of the $Al_2O_3$, $SiO_2$, and $M_{2/n}O$ components, respectively, in the alumina-silica-metal oxide combination, with a being from greater than 0 to 100, b being 0 to 100, and c being 0 to 50, and n is an integer from 1 to 7 of the valence of the metal cation, and

(b) a catalytically effective amount of sulfur trioxide, the said catalyst composition containing not more than 5 weight percent, based on the weight of the alumina-silica-metal oxide combination, of other substances.

2. The catalyst composition of Claim 1 wherein the amount of sulfur trioxide is in the range from about 5 weight percent to about 40 weight percent, based on the weight of the alumina-silica-metal oxide combination.

3. The catalyst composition of Claim 1 wherein the alumina-silica-metal oxide combination is selected from crystalline and non-crystalline phases, and mixtures thereof.

4. A process for preparing a catalyst composition which comprises contacting under substantially anhydrous conditions an alumina-silica-metal oxide combination represented by the formula

$$(Al_2O_3)_a(SiO_2)_b(M_{2/n}O)_c$$

wherein M is a metal cation selected from the lanthanides, Groups 1b, 2b, 5b, 6b, 7b, and 8 of the Periodic Table of the Elements, and mixtures thereof, and a, b, and c represent weight percent of the $Al_2O_3$, $SiO_2$, and $M_{2/n}O$ components, respectively, in the alumina-silica-metal oxide combination, with a being from greater than 0 to 100, b being 0 to 100, and c being 0 to 50, and n is an integer from 1 to 7 of the valence of the cation, the said combination containing not more than 5 weight percent of other substances, with a catalytically effective amount of gaseous sulfur trioxide.

5. The process of Claim 4 wherein the effective amount of sulfur trioxide is in the range from about 5 weight percent to about 40 weight percent, based on the weight of the alumina-silica-metal oxide combination.

6. The process of Claim 4 wherein the contacting of the alumina-silica-metal oxide combination with the sulfur trioxide is carried out at a temperature from about 25°C. to about 300°C.

7. The process of Claim 6 wherein the temperature is from about 150°C. to about 225°C.

8. The process of Claim 7 wherein the temperature is about 175°C.

9. The process of Claim 4 wherein the sulfur trioxide is provided by contacting the alumina-silica-metal oxide combination with a mixture of sulfur dioxide and nitrogen dioxide.

10. The process of Claim 9 wherein the mole ratio of sulfur dioxide to nitrogen dioxide is at least 1.

11. The process of Claim 10 wherein the mole ratio of sulfur dioxide to nitrogen dioxide is about 2—3/l.

12. A process for the vapor phase nitration of aromatic compounds where the aromatic compound is contacted with a nitrating agent in the vapor phase to yield the corresponding nitroaromatic compound, characterised in that the nitration is conducted in the presence of water and a nitration promoting catalyst which comprises the adduct of:

(a) an alumina-silica-metal oxide combination represented by the formula:

$$(Al_2O_3)_a(SiO_2)_b(M_2/_nO)_c$$

wherein M is a metal cation selected from the group consisting of the lanthanides or rare earths, Groups 1b, 2b, 5b, 6b, 7b, and 8 of the Periodic Table of the Elements, and mixtures thereof, and a, b, and c represent weight percent of the $Al_2O_3$, $SiO_2$, and $M_{2/n}O$ components, respectively, in the alumina-silica-metal oxide combination, with a being 0 to 100, b being 0 to 100, and c being 0 to 50, and n represents an integer from 1 to 7 of the valence of the metal cation, with the proviso that the sum of (a+b) must be greater than 0, and

(b) a catalytically effective amount of sulfur trioxide.

13. The process of claim 12 wherein the amount of sulfur trioxide is in the range from about 5 weight percent to about 40 weight percent, based on the weight of the alumina-silica-metal oxide combination.

14. The process of Claim 12 wherein the alumina-silica-metal oxide combination is selected from the group consisting of crystalline and noncrystalline phases, and mixtures thereof.

15. The process of Claim 12 wherein the nitrating agent is nitrogen dioxide.

16. The process of Claim 12 wherein the nitrating agent is admixed with a carrier gas prior to reaction with the aromatic compound.

17. The process of Claim 16 wherein the carrier gas is nitrogen.

18. The process of Claim 12 wherein the nitration promoting catalyst is conditioned by pretreatment with the nitrating agent.

19. The process of Claim 18 wherein the pretreatment is carried out for about 1 minute to about 1 hour.

20. The process of Claim 12 wherein the aromatic compound is an aromatic hydrocarbon.

21. The process of Claim 20 wherein the aromatic hydrocarbon is selected from the group consisting of benzene and toluene.

22. The process of Claim 12 wherein the aromatic compound is a haloaromatic compound.

23. The process of Claim 22 wherein the haloaromatic compound is selected from the group consisting of chlorobenzene, bromobenzene, iodobenzene, and o-dichlorobenzene.

24. The process of Claim 12 wherein the aromatic compound is an aromatic ether.

25. The process of Claim 24 wherein the aromatic ether is selected from anisole and phenetole.

26. The process of Claim 12 wherein the aromatic compound is an aromatic carboxylate.

27. The process of Claim 13 wherein the aromatic carboxylate is selected from the group consisting of benzoic acid, methyl benzoate, and ethyl benzoate.

28. The process of Claim 12 wherein the concentration of the aromatic compound in the feed mixture is between about 1 percent and about 15 percent by volume.

29. The process of Claim 12 wherein about 0.5 to about 5 moles of nitrating agent are used per mole of aromatic compound.

30. The process of Claim 12 wherein the aromatic compound is admixed with a carrier gas prior to reaction with the nitrating agent.

31. The process of Claim 30 wherein the carrier gas is an oxygen-containing gas.

32. The process of Claim 31 wherein the oxygen-containing gas is air.

33. The process of Claim 12 wherein water vapor is admixed with the feed mixture prior to reaction between the aromatic compound and the nitrating agent.

34. The process of Claim 33 wherein the water vapor is present in the feed mixture in a concentration ranging from about 0.1 percent to about 15 percent by volume.

35. The process of Claim 12 wherein the vapor phase reaction is carried out at temperatures ranging from about 80°C. to about 300°C.

36. The process of Claim 35 wherein the temperature ranges from about 150°C. to about 250°C.

37. The process of Claim 12 wherein the aromatic compound is a monosubstituted aromatic compound having an ortho-para orientation substituent and the nitroaromatic compound is a mixture of ortho, meta, and para isomers.

38. The process of Claim 37 wherein the monosubstituted aromatic compound is chlorobenzene and the nitroaromatic compound is a mixture of o-, m-, and p-nitrochlorobenzene.

**Patentansprüche**

1. Katalysatorzubereitung enthaltend das Addukt aus

(a) einer Aluminiumoxid-Siliciumdioxid-Metalloxid-Kombination der Formel

$$(Al_2O_3)_a(SiO_2)_b(M_{2/n}O)_c$$

in der M für ein Metallkation ausgewählt aus den Lanthaniden, den Gruppen 1b, 2b, 5b, 6b, 7b und 8 des Periodensystems der Elemente und Mischungen davon steht und a, b und c den Gewichtsprozentsatz der Bestandteile $Al_2O_3$, $SiO_2$ bzw. $M_{2/n}O$ in der Aluminiumoxid-Siliciumdioxid-Metalloxid-Kombination repräsentieren, wobei a einen Wert von mehr als 0 bis 100, b einen Wert von 0 bis 100 und c einen Wert von 0 bis 50 aufweisen und n für eine ganze Zahl mit einem Wert von 1 bis 7, die der Wertigkeit des Metallkations entspricht, steht, und

(b) einer katalytisch wirksamen Menge Schwefeltrioxid, wobei die Katalysatorzubereitung nicht mehr als 5 Gew.-% anderer Substanzen, bezogen auf das Gewicht aus der Aluminiumoxid-Siliciumdioxid-Metalloxid-Kombination enthält.

2. Katalysatorzubereitung nach Anspruch 1, dadurch gekennzeichnet, daß die Menge des Schwefeltrioxids im Bereich von etwa 5 Gew.-% bis etwa 40 Gew.-%, bezogen auf das Gewicht der Aluminiumoxid-Siliciumdioxid-Metalloxid-Kombination, liegt.

3. Katalysatorzubereitung nach Anspruch 1, dadurch gekennzeichnet, daß die Aluminiumoxid-Siliciumdioxid-Metalloxid-Kombination aus kristallinen Phasen, nichtkristallinen Phasen und Mischungen davon ausgewählt ist.

4. Verfahren zur Herstellung einer Katalysatorzubereitung, dadurch gekennzeichnet, daß man unter im wesentlichen wasserfreien Bedingungen eine Aluminiumoxid-Siliciumdioxid-Metalloxid-Kombination der Formel

$$(Al_2O_3)_a(SiO_2)_b(M_{2/n}O)_c$$

in der M für ein Metallkation, ausgewählt aus den Lanthaniden, den Gruppen 1b, 2b, 5b, 6b, 7b und 8 des Periodensystems der Elemente und Mischungen davon steht und a, b und c den Gewichtsprozentsatz der Bestandteile $Al_2O_3$, $SiO_2$ bzw. $M_{2/n}O$ in der Aluminiumoxid-Siliciumdioxid-Metalloxid-Kombination repräsentieren, wobei a einen Wert von mehr als 0 bis 100, b einen Wert von 0 bis 100 und c einen Wert von 0 bis 50 aufweisen, und n für eine ganze Zahl mit einem Wert von 1 bis 7, die der Wertigkeit des Metallkations entspricht, steht, welche Kombination nicht mehr als 5 Gew.-% anderer Substanzen enthält, mit einer katalytisch wirksamen Menge gasförmigen Schwefeltrioxids in Kontakt bringt.

5. Verfahren nach Anspruch 4, dadurch gekennzeichnet, daß die wirksame Menge des Schwefeltrioxids im Bereich von etwa 5 Gew.-% bis etwa 40 Gew.-%, bezogen auf das Gewicht der Aluminiumoxid-Siliciumdioxid-Metalloxid-Kombination, liegt.

6. Verfahren nach Anspruch 4, dadurch gekennzeichnet, daß man die Aluminiumoxid-Siliciumdioxid-Metalloxid-Kombination bei einer Temperatur von etwa 25°C bis etwa 300°C mit dem Schwefeltrioxid in Kontakt bringt.

7. Verfahren nach Anspruch 6, dadurch gekennzeichnet, daß die Temperatur etwa 150°C bis etwa 225°C beträgt.

8. Verfahren nach Anspruch 7, dadurch gekennzeichnet, daß die Temperatur etwa 175°C beträgt.

9. Verfahren nach Anspruch 4, dadurch gekennzeichnet, daß das Schwefeltrioxid dadurch zugeführt wird, daß man die Aluminiumoxid-Siliciumdioxid-Metalloxid-Kombination mit einer Mischung aus Schwefeldioxid und Stickstoffdioxid in Kontakt bringt.

10. Verfahren nach Anspruch 9, dadurch gekennzeichnet, daß das Molverhältnis von Schwefeldioxid zu Stickstoffdioxid mindestens 1 beträgt.

11. Verfahren nach Anspruch 10, dadurch gekennzeichnet, daß das Molverhältnis von Schwefeldioxid zu Stickstoffdioxid etwa 2 bis 3/l beträgt.

12. Verfahren zur Dampfphasen-Nitrierung von aromatischen Verbindungen, bei dem die aromatische Verbindung in der Dampfphase unter Bildung der entsprechenden nitroaromatischen Verbindung mit einem Nitrierungsmittel in Kontakt gebracht wird, dadurch gekennzeichnet, daß man die Nitrierung in Gegenwart von Wasser und eines die Nitrierung fördernden Katalysators durchführt, der das Addukt aus:

(a) einer Aluminiumoxid-Siliciumdioxid-Metalloxid-Kombination der Formel

$$(Al_2O_3)_a(SiO_2)_b(M_{2/n}O)_c$$

in der M für ein Metallkation, ausgewählt aus den Lanthaniden, den Gruppen 1b, 2b, 5b, 6b, 7b und 8 des Periodensystems der Elemente und Mischungen davon steht und a, b und c den Gewichtsprozentsatz der Bestandteile $Al_2O_3$, $SiO_2$ bzw. $M_{2/n}O$ in der Aluminiumoxid-Siliciumdioxid-Metalloxid-Kombination repräsentieren, wobei a einen Wert von mehr als 0 bis 100, b einen Wert von 0 bis 100 und c einen Wert von 0 bis 50 aufweisen und n für eine ganze Zahl mit einem Wert von 1 bis 7, die der Wertigkeit des Metallkations entspricht, steht, mit der Maßgabe, daß die Summe (a+b) größer als 0 sein muß, und

(b) einer katalytisch wirksamen Menge Schwefeltrioxid umfaßt.

13. Verfahren nach Anspruch 12, dadurch gekennzeichnet, daß die Schwefeltrioxidmenge im Bereich von etwa 5 Gew.-% bis etwa 40 Gew.-%, bezogen auf das Gewicht der Aluminiumoxid-Siliciumdioxid-Metalloxid-Kombination, liegt.

14. Verfahren nach Anspruch 12, dadurch gekennzeichnet, daß die Aluminiumoxid-Siliciumdioxid-Metalloxid-Kombination aus der Gruppe ausgewählt ist, die kristalline Phasen, nichtkristalline Phasen und Mischungen davon umfaßt.

15. Verfahren nach Anspruch 12, dadurch gekennzeichnet, daß man als Nitrierungsmittel Stickstoffdioxid verwendet.

16. Verfahren nach Anspruch 12, dadurch gekennzeichnet, daß man das Nitrierungsmittel vor der Reaktion mit der aromatischen Verbindung mit einem Trägergas vermischt.

17. Verfahren nach Anspruch 16, dadurch gekennzeichnet, daß man als Trägergas Stickstoff verwendet.

18. Verfahren nach Anspruch 12, dadurch gekennzeichnet, daß man den die Nitrierung fördernden Katalysator durch Vorbehandeln mit dem Nitrierungsmittel konditioniert.

19. Verfahren nach Anspruch 18, dadurch gekennzeichnet, daß die Vorbehandlung während etwa 1 Minute bis etwa 1 Stunde durchgeführt wird.

20. Verfahren nach Anspruch 12, dadurch gekennzeichnet, daß die aromatische Verbindung ein aromatischer Kohlenwasserstoff ist.

21. Verfahren nach Anspruch 20, dadurch gekennzeichnet, daß der aromatische Kohlenwasserstoff aus der Benzol und Toluol umfassenden Gruppe ausgewählt ist.

22. Verfahren nach Anspruch 12, dadurch gekennzeichnet, daß die aromatische Verbindung eine halogenaromatische Verbindung ist.

23. Verfahren nach Anspruch 22, dadurch gekennzeichnet, daß die halogenaromatische Verbindung aus der Gruppe ausgewählt ist, die Chlorbenzol, Brombenzol, Iodbenzol und o-Dichlorbenzol umfaßt.

24. Verfahren nach Anspruch 12, dadurch gekennzeichnet, daß die aromatische Verbindung ein aromatischer Ether ist.

25. Verfahren nach Anspruch 24, dadurch gekennzeichnet, daß der aromatische Ether aus Anisol und Phenetol ausgewählt ist.

26. Verfahren nach Anspruch 12, dadurch gekennzeichnet, daß die aromatische Verbindung ein aromatisches Carboxylat ist.

27. Verfahren nach Anspruch 13, dadurch gekennzeichnet, daß das aromatische Carboxylat aus der Gruppe ausgewählt ist, die Benzoseäure, Benzoesäuremethylester und Benzoesäuremethylester und Benzoesäureethylester umfaßt.

28. Verfahren nach Anspruch 12, dadurch gekennzeichnet, daß die Konzentration der aromatischen Verbindung in der Beschickungsmischung zwischen etwa 1 Vol.-% und etwa 15 Vol.-% liegt.

29. Verfahren nach Anspruch 12, dadurch gekennzeichnet, daß man etwa 0,5 bis etwa 5 Mol des Nitrierungsmittels pro Mol der aromatischen Verbindung verwendet.

30. Verfahren nach Anspruch 12, dadurch gekennzeichnet, daß man die aromatische Verbindung vor der Umsetzung mit dem Nitrierungsmittel mit einem Trägergas vermischt.

31. Verfahren nach Anspruch 30, dadurch gekennzeichnet, daß das Trägergas ein sauerstoffhaltiges Gas ist.

32. Verfahren nach Anspruch 31, dadurch gekennzeichnet, daß das sauerstoffhaltige Gas Luft ist.

33. Verfahren nach Anspruch 12, dadurch gekennzeichnet, daß man die Beschickungsmischung vor der Reaktion zwischen der aromatischen Verbindung und dem Nitrierungsmittel mit Wasserdampf vermischt.

34. Verfahren nach Anspruch 33, dadurch gekennzeichnet, daß der Wasserdampf in der Beschickungsmischung in einer Konzentration vorhanden ist, die sich von etwa 0,1 Vol.-% bis etwa 15 Vol.-% erstreckt.

35. Verfahren nach Anspruch 12, dadurch gekennzeichnet, daß man die Dampfphasenreaktion bei Temperaturen im Bereich von etwa 80 bis etwa 300°C durchführt.

36. Verfahren nach Anspruch 35, dadurch gekennzeichnet, daß die Temperatur im Bereich von etwa 150°C bis etwa 250°C liegt.

37. Verfahren nach Anspruch 12, dadurch gekennzeichnet, daß die aromatische Verbindung eine monosubstituierte aromatische Verbindung mit einem Substituenten in einer ortho-para-Stellung und die nitroaromatische Verbindung eine Mischung aus den ortho-, meta- und para-Isomeren sind.

38. Verfahren nach Anspruch 37, dadurch gekennzeichnet, daß die monosubstituierte aromatische Verbindung Chlorbenzol und die nitroaromatische Verbindung eine Mischung aus o-, m- und p-Nitrochlorbenzol sind.

**Revendications**

1. Composition de catalyseur comprenant le produit d'addition:

(a) d'une combinaison alumine-silice-oxyde métallique répondant à la formule:

$$(Al_2O_3)_a(SiO_2)_b(M_{2/n}O)_c$$

dans laquelle M est un cation métallique choisi parmi les lanthanides, les groupes 1b, 2b, 5b, 6b, 7b et 8 de la classification Périodique des Eléments, et des mélanges de ceux-ci et a, b et c représentent des pourcentages pondéraux de $Al_2O_3$, $SiO_2$ et $M_{2/n}O$, respectivement, dans la combinaison alumine-silice-oxyde métallique, a étant supérieur à O et allant jusqu'à 100, b étant un nombre de 0 à 100, et c étant un nombre de 0 à 50, et n est un entier de 1 à 7 égal à la valence du cation métallique, et

(b) d'une quantité catalytiquement efficace d'anhydride sulfurique, cette composition de catalyseur ne contenant pas plus de 5% en poids, par rapport au poids de la combinaison alumine-silice-oxyde métallique, d'autres substances.

2. Composition de catalyseur selon la revendication 1, dans laquelle le pourcentage d'anhydride sulfurique est dans l'intervalle d'environ 5% à environ 40% en poids, par rapport au poids de la combinaison alumine-silice-oxyde métallique.

3. Composition de catalyseur selon la revendication 1, dans laquelle la combinaison alumine-silice-oxyde métallique est choisie parmi des phases cristallines et non cristallines, et des mélanges de celles-ci.

4. Procédé pour préparer une composition de catalyseur qui consiste à mettre en contact, dans des conditions pratiquement anhydres, une combinaison alumine-silice-oxyde métallique répondant à la formule:

$$(Al_2O_3)_a(SiO_2)_b(M_{2/n}O)_c$$

dans laquelle M est un cation métallique choisi parmi les lanthanides, les groupes 1b, 2b, 5b, 6b, 7b et 8 de la Classification Périodique des Eléments, et des mélanges de ceux-ci, et a, b et c représentant des pourcentages pondéraux d'$Al_2O_3$, $SiO_2$ et $M_{2/n}O$, respectivement dans la combinaison alumine-silice-oxyde métallique, a étant un nombre supérieur à 0 et allant jusqu'à 100, b étant un nombre de 0 à 100, et c étant un nombre de 0 à 50 et n étant un entier de 1 à 7 égal à la valence du cation, cette combinaison ne contenant pas plus de 5% en poids d'autres substances, avec une quantité catalytiquement efficace d'anhydride sulfurique gazeux.

5. Procédé selon la revendication 4, dans lequel la quantité efficace d'anhydride sulfurique est dans l'intervalle d'environ 5% en poids à environ 40% en poids, par rapport au poids de la combinaison alumine-silice-oxyde métallique.

6. Procédé selon la revendication 4, dans lequel la mise en contact de la combinaison alumine-silice-oxyde métallique avec l'anhydride sulfurique est effectuée à une température d'environ 25 à environ 300°C.

7. Procédé selon la revendication 6, dans lequel la température est d'environ 150 à environ 225°C.

8. Procédé selon la revendication 7, dans lequel la température est d'environ 175°C.

9. Procédé selon la revendication 4, dans lequel l'anhydride sulfurique est obtenu en mettant en contact la combinaison alumine-silice-oxyde métallique avec un mélange d'anhydride sulfureux et de bioxyde d'azote.

10. Procédé selon la revendication 9, dans lequel le rapport molaire de l'anhydride sulfureux au bioxyde d'azote est d'au moins 1.

11. Procédé selon la revendication 10, dans lequel le rapport molaire de l'anhydride sulfureux au bioxyde d'azote est d'environ 2—3/l.

12. Procédé pour la nitration en phase vapeur de composés aromatiques, dans lequel le composé aromatique est mis en contact avec un agent nitrant en phase vapeur pour donner le composé nitroaromatique correspondant, caractérisé en ce que la nitration est effectuée en présence d'eau et d'un catalyseur d'activation de la nitration qui comprend le produit d'addition:

(a) d'une combinaison alumine-silice-oxyde métallique répondant à la formule:

$$(Al_2O_3)_a(SiO_2)_b(M_{2/n}O)_c$$

dans laquelle M est un cation métallique choisi dans le groupe constitué des lanthanides ou des terres rares, des groupes 1b, 2b, 5b, 6b, 7b et 8 de la Classification Périodique des Eléments, et de mélanges de ceux-ci, et a, b et c représentent des pourcentages pondéraux d'$Al_2O_3$, $SiO_2$ et $M_{2/n}O$ respectivement, dans la combinaison alumine-silice-oxyde métallique, a étant un nombre de 0 à 100, b étant un nombre de 0 à 100 et c étant un nombre de 0 à 50, et n représentant un entier de 1 à 7 égal à la valence du cation métallique, sous réserve que la somme (a+b) soit supérieure à 0, et

(b) d'une quantité catalytiquement efficace d'anhydride sulfurique.

13. Procédé selon la revendication 12, dans lequel la quantité d'anhydride sulfurique est dans l'intervalle d'environ 5% en poids à environ 40% en poids, par rapport au poids de la combinaison alumine-silice-oxyde métallique.

14. Procédé selon la revendication 12, dans lequel la combinaison alumine-silice-oxyde métallique est choisie dans le groupe constitué des phases cristallines et non-cristallines et de mélanges de celles-ci.

15. Procédé selon la revendication 12, dans lequel l'agent de nitration est le bioxyde d'azote.

16. Procédé selon la revendication 12, dans lequel l'agent de nitration est mélangé avec un gaz porteur avant la réaction avec le composé aromatique.

17. Procédé selon la revendication 16, dans lequel le gaz porteur est l'azote.

18. Procédé selon la revendication 12, dans lequel le catalyseur activant la nitration est conditionné par pré-traitement avec l'agent nitrant.

19. Procédé selon la revendication 18, dans lequel le pré-traitement est effectué pendant environ 1 minute à environ 1 heure.

20. Procédé selon la revendication 12, dans lequel le composé aromatique est un hydrocarbure aromatique.

21. Procédé selon la revendication 20, dans lequel l'hydrocarbure aromatique est choisi dans le groupe constitué du benzène et du toluène.

22. Procédé selon la revendication 12, dans lequel le composé aromatique est un composé haloaromatique.

23. Procédé selon la revendication 22, dans lequel le composé haloaromatique est choisi dans le groupe constitué du chlorobenzène, du bromobenzène, de l'iodobenzène et del'orthodichlorobenzène.

24. Procédé selon la revendication 12, dans lequel le composé aromatique est un éther aromatique.

25. Procédé selon la revendication 24, dans lequel l'éther aromatique est choisi parmi l'anisole et le phénétole.

26. Procédé selon la revendication 12, dans lequel le composé aromatique est un carboxylate aromatique.

27. Procédé selon la revendication 13, dans lequel le carboxylate aromatique est choisi dans le groupe constitué de l'acide benzoïque, du benzoate de méthyle et du benzoate d'éthyle.

28. Procédé selon la revendication 12, dans lequel la concentration du composé aromatique dans le mélange d'alimentation est entre environ 1% et environ 15% en volume.

29. Procédé selon la revendication 12, dans lequel environ 0,5 à environ 5 moles d'agent nitrant sont utilisés par mole de composé aromatique.

30. Procédé selon la revendication 12, dans lequel le composé aromatique est mélangé à un gaz porteur avant la réaction avec l'agent nitrant.

31. Procédé selon la revendication 30, dans lequel le gaz porteur est un gaz oxygéné.

32. Procédé selon la revendication 31, dans lequel le gaz oxygéné est l'air.

33. Procédé selon la revendication 12, dans lequel de la vapeur d'eau est mélangée au mélange d'alimentation avant la réaction entre le composé aromatique et l'agent nitrant.

34. Procédé selon la revendication 33, dans lequel de la vapeur d'eau est présente dans le mélange d'alimentation, à une concentration allant d'environ 0,1% à environ 15% en volume.

35. Procédé selon la revendication 12, dans lequel la réaction en phase vapeur est effectuée à des températures allant d'environ 80°C à environ 300°C.

36. Procédé selon la revendication 35, dans lequel la température va d'environ 150°C à environ 250°C.

37. Procédé selon la revendication 12, dans lequel le composé aromatique est un composé aromatique monosubstitué ayant un substituant orientant en ortho- para et le composé nitroaromatique est un mélange d'isomères ortho, méta et para.

38. Procédé selon la revendication 37, dans lequel le composé aromatique monosubstitué est du chlorobenzène et le composé nitroaromatique est un mélange de o-, de m- et de p-nitrochlorobenzène.